(19) Europäisches Patentamt / European Patent Office / Office européen des brevets

(11) EP 2 647 638 A1

(12) **EUROPEAN PATENT APPLICATION**

(43) Date of publication:
**09.10.2013 Bulletin 2013/41**

(21) Application number: **12382131.6**

(22) Date of filing: **02.04.2012**

(51) Int Cl.:
***C07D 491/04*** (2006.01) ***A61K 31/4355*** (2006.01)
***A61K 31/436*** (2006.01) ***A61P 1/00*** (2006.01)
***A61P 7/00*** (2006.01) ***A61P 9/00*** (2006.01)
***A61P 13/00*** (2006.01) ***A61P 19/00*** (2006.01)
***A61P 25/00*** (2006.01) ***A61P 31/00*** (2006.01)
***A61P 35/00*** (2006.01) ***A61P 37/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Almirall, S.A.**
  **08022 Barcelona (ES)**
- **Draconis Pharma, S.L.**
  **08225 Terrassa - Barcelona (ES)**
- **Laboratorios del. Dr. Esteve, S.A.**
  **08041 Barcelona (ES)**

(72) Inventors:
- **Aguilar, Nuria**
  **08005 Barcelona (ES)**
- **Fernandez, Joan, Carles**
  **08004 Barcelona (ES)**
- **Terricabras, Emma**
  **08173 Sant Cugat del Valles (Barcelona) (ES)**
- **Carceller Gonzalez, Elena**
  **08173 Sant Cugat del Valles (Barcelona) (ES)**
- **Garcia Garcia, Francisco Javier**
  **08921 Santa Coloma de Gramanet (Barcelona) (ES)**

(74) Representative: **Carpintero Lopez, Francisco et al**
**Herrero & Asociados, S.L.**
**Alcalá 35**
**28014 Madrid (ES)**

(54) **Substituted tricyclic compounds with activity towards ep1 receptors**

(57) The present invention belongs to the field of EP1 receptor ligands. More specifically it refers to compounds of general formula (I) having great affinity and selectivity for the EP1 receptor. The invention also refers to the process for their preparation, to their use as medicament for the treatment and/or prophylaxis of diseases or disorders mediated by the EP1 receptor as well as to pharmaceutical compositions comprising them.

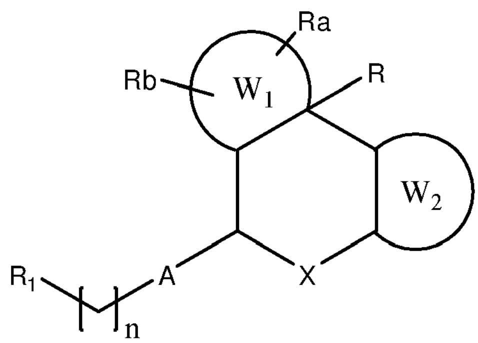

**(I)**

EP 2 647 638 A1

## Description

### FIELD OF THE INVENTION

The present invention belongs to the field of EP1 receptor ligands. More specifically it refers to compounds of general formula (I) having great affinity and selectivity for the EP1 receptor. The invention also refers to the process for their preparation, to their use as medicament for the treatment and/or prophylaxis of diseases or disorders mediated by the EP1 receptor as well as to pharmaceutical compositions comprising them.

### BACKGROUND OF THE INVENTION

Prostanoids are a family of eicosanoids that comprise prostaglandins (PGs), prostacyclins (PGIs), and thromboxanes (Txs) . Their receptors belong to the G- protein coupled receptor (GPCR) superfamily of receptors and may be grouped into five classes, namely, prostaglandin D (DP), prostaglandin E (EP), prostaglandin F (FP), prostaglandin I (IP), and Thromboxane A (TP) based on their sensitivity to five naturally occurring prostanoids, PGD2, PGE2, PGF2 [alpha], PGI2, and TxA2, respectively (Coleman, R. A., 2000) .

Prostaglandins contribute to the sensitization of peripheral and central nociceptive neurons during peripheral inflammation (Dirig and Yaksh, 1999) and play an important role in the pathogenesis of neuropathic pain following nerve injury (Syriatowicz et al 1999; Kawahara et al, 2001; Samad et al, 2002; Ma and Eisenach, 2003; Durrenberger et al., 2006).

Prostaglandin E2 (PGE2) is considered to be the dominant pro-nociceptive prostanoid. Guay and colleagues, analyzing the concentrations of different prostaglandins in the cerebrospinal fluid, found that PGE2 was the most prevalent prostanoid and exhibited the highest increase after peripheral carrageenan-induced inflammation (Guay et al., 2004). PGE2 is generated in most cells in response to mechanical, thermal or chemical injury and inflammatory insult, resulting in sensitization or direct activation of nearby sensory nerve endings. Its production requires the activity of at least one of the two cyclooxygenase isoforms, COX-1 constitutively expressed or COX-2 which is inducible and particularly relevant for inflammation-induced PGE2 formation. Therefore, non-selective inhibitors of COX-1 and COX-2, and selective COX-2 inhibitors provide good pain relief. However, the long-term use is associated with gastrointestinal or cardiovascular side effects, respectively.

Downstream components of the inflammatory cascade could be an alternative approach for the treatment of the PGE2 associated pain. PGE2 binds to four different G-protein coupled receptors named EP1, EP2, EP3 and EP4 (Narumiya et al., 1999).

Studies employing antagonists suggest that blocking EP1, EP2, EP3 or EP4 receptors may reduce certain types of pain (Oka et al. 1997; Omote et al., 2002; Lin et al, 2006) and agonists increase nociceptive responses (Minami et al., 1994). Among these PGE2 receptor subtypes, most of drug discovery studies have focused on the EP1 receptors (Hall et al., 2007).

EP1 receptor stimulation mediates increases in intracellular calcium ions, facilitating neurotransmitter release (Asbóth et al., 1996). EP1 receptor is preferentially expressed in primary sensory neurons, including their spinal cord terminals (Oidda et al., 1995) although it is also distributed in other tissues (Breyer et al., 2000; Schlötzer-Schrehardt et al., 2002). In the brain, marked differences in the level of EP1 expression among the cerebral regions were found. The strongest levels of EP1 mRNA were found in parietal cortex and cerebellum, followed in descending order by frontal cortex and striatum. The hypothalamus, hippocampus and brain stem displayed a low-level EP1 mRNA signal (Candelario-Jalil et al., 2005). In the spinal cord, several studies have reported the effects of PGE2 on neuronal excitability or synaptic transmission (Baba et al., 2001) and pain transmission (Nakayama et al., 2004). Therefore, EP1 receptor antagonists, blocking the positive feedback cascade mediated by $PGE_2$, may result in analgesic efficacy. In this regard, using EP receptor deficient mice, a prominent contribution of EP1 receptors has been described (Minami et al., 2001). EP1 -/- knockout mice demonstrated a role of this receptor in mediating peripheral heat sensitization after subcutaneous PGE2 injection (Moriyama et al. 2005; Johansson et al. 2011), and EP1 receptor antagonism has been reported to reduce mechanical hyperalgesia in nerve injured rats (Kawahara et al., 2001), in the carrageenan model (Nakayama et al. 2002), or in the incisional model of postoperative pain (Omote et al 2002). Moreover, EP1 antagonists demonstrated analgesic activity in a complete Freund's adjuvant model of knee joint arthritis (Giblin et al, 2007; Hall et al, 2009). It has also been reported that the contribution of PGE2 in human visceral pain hypersensitivity is mediated through the EP1 receptor (Sarkar et al., 2003).

In addition to being useful for modulating pain, EP1 antagonists may also be useful for the treatment or prevention of other EP1 receptor-mediated diseases such as motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of

fever as rheumatic fever; symptoms associated with influenza or other viral infections; gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases.

In turn, EP1 receptor agonists also may have a number of utilities. These include, but are not limited to treatment of influenza, bone fracture healing, bone disease, glaucoma, ocular hypertension, dysmenorrhoea, pre-term labour, immune disorders, osteoporosis, asthma, allergy, fertility, male sexual dysfunction, female sexual dysfunction, periodontal disease, gastric ulcer, and renal disease. EP receptor agonists may also be useful for expansion of hematopoietic stem cell populations.

Based on the above mentioned results coming from animal and human studies, EP1 receptor has been identified as a selective target for the development of new potential therapies for the treatment of those disorders where PGE2 action is involved. In view of the potential therapeutic applications of agonists and antagonists of the EP1 receptor, a great effort is being directed to find selective ligands. Despite intense research efforts in this area, very few compounds with selective EP1 activity have been reported.

There is thus still a need to find compounds having pharmacological activity towards the EP1 receptor, being both effective and selective, and having good "drugability" properties, i.e. good pharmaceutical properties related to administration, distribution, metabolism and excretion.

The present invention hereby provide some novel compounds complying with the above mentioned properties.

OBJECT OF THE INVENTION

The present invention discloses novel compounds with great affinity to EP1 receptors which might be used for the treatment of EP1-related disorders or diseases.

Specifically, it is an object of the invention a compound of general formula I:

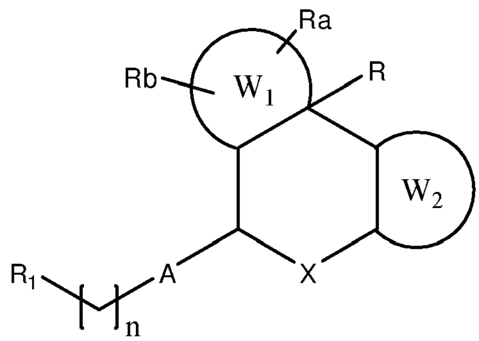

**(I)**

wherein:

W1 is a 5or 6-membered saturated heterocyclic ring containing 1 or 2 heteroatoms selected from N, O or S;
W2 is a benzene ring optionally substituted by one or more $R_2$ and optionally fused to a carbocyclic ring; or a 5 or 6-membered heterocyclic ring containing 1 or 2 heteroatoms selected from N, O or S, optionally substituted by one or more $R_2$;
**R** is a hydrogen; or $C_{1-6}$- alkyl;
**Ra** and **Rb** are independently selected from hydrogen, =O;- OH;- $CH_2OH$;- CO- $C_{1-6}$- alkyl;- $SO_2$- $C_{1-6}$- alkyl; $C_{1-6}$-alkyl;- O- $C_{1-6}$- alkyl or- O- $C_{1-6}$- haloalkyl;

**$R_1$** is a- COOH; a tetrazol; a- $SO_2$- NH- C (=O)- $R_3$; a- C (=O) NH- $SO_2$- $R_4$; a- $SO_2$- OH or a- CN;
**$R_2$** is independently selected from hydrogen; halogen; $C_{1-6}$ alkyl; C1-$_6$- haloalkyl;- O- $C_{1-6}$- alkyl;- O- $C_{1-6}$- haloalkyl; $C_{3-6}$cycloalkyl;- O- $C_{3-6}$cycloalkyl;- $NR_5SO_2R_6$; phenyl;- $CONH_2$ or- CN;
**$R_3$** and **$R_4$** are independently selected from a hydrogen; $C_{1-6}$- alkyl; an optionally substituted phenyl; or- N $(CH_3)_2$;
**$R_5$** and **$R_6$** are independently selected from a hydrogen; or $C_{1-6}$-alkyl
**$R_7$** is selected from a hydrogen; $C_{1-6}$- alkyl; or a- $COR_8$
**$R_8$** is a $C_{1-6}$- alkyl;
**X** is selected from $NR_7$ or O;
**A** is a phenyl optionally substituted by one or more substituents selected from $C_{1-6}$- alkyl, halogen, $C_{1-6}$- haloalkyl,

-O- $C_{1-6}$- alkyl, , -O- $C_{1-6}$- haloalkyl, -NH- CO- $CH_3$, a- CN or a $C_{3-6}$cycloalkyl optionally substituted a $C_{1-6}$- alkyl, ; or an heteroaromatic ring or ring system optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted a $C_{1-6}$- alkyl, ;

n is 0, 1 or 2,

and the salts, solvates and prodrugs thereof.

It is also an object of the invention the process for the preparation of compounds of general formula (I).

In another aspect, the invention relates to a compound of general formula (I) for use as a medicament.

Yet another object of the invention is a compound of general formula (I) for use in the treatment and/or prophylaxis of diseases or disorders mediated by the EP1 receptor. This includes but is not limited to diseases such as inflammatory related pain including low back and neck pain, skeletal pain, post-partum pain, toothache, sprains and straits, myositis, neuralgia, synovitis, arthritis, including rheumatoid arthritis, degenerative joint diseases, gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases.

It is another object of the invention a pharmaceutical composition comprising at least one compound of general formula (I) and at least one pharmaceutically acceptable carrier, additive, adjuvant or vehicle.

## DETAILED DESCRIPTION OF THE INVENTION

In a first aspect the invention relates to compounds of general formula (I):

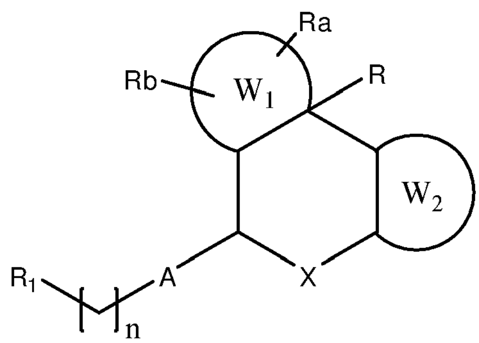

**(I)**

wherein:

**W1** is a 5 or 6-membered saturated heterocyclic ring containing 1 or 2 heteroatoms selected from N, O or S;

**W2** is a benzene ring optionally substituted by one or more $R_2$ and optionally fused to a carbocyclic ring; or a 5 or 6-membered heterocyclic ring containing 1 or 2 heteroatoms selected from N, O or S, optionally substituted by one or more $R_2$;

**R** is a hydrogen; or $C_{1-6}$-alkyl

**Ra** and **Rb** are independently selected from hydrogen; =O;- OH;- $CH_2OH$;- CO- $C_{1-6}$- alkyl;- $SO_2$- $C_{1-6}$- alkyl; $C_{1-6}$- alkyl;- O- $C_{1-6}$- alkyl or- O- $C_{1-6}$- haloalkyl;

**$R_1$** is a- COOH; a tetrazol; a- $SO_2$- NH- C (=O)- $R_3$; a- C (=O) NH- $SO_2$- $R_4$; a- $SO_2$- OH or a- CN;

**$R_2$** is independently selected from hydrogen; halogen; $C_{1-6}$ alkyl; $C_{1-6}$- haloalkyl;- O- $C_{1-6}$- alkyl;- O- $C_{1-6}$- haloalkyl; $C_{3-6}$cycloalkyl;- O- $C_{3-6}$cycloalkyl;- $NR_5SO_2R_6$;

phenyl; $-CONH_2$ or -CN;
**$R_3$** and **$R_4$** are independently selected from a hydrogen; $C_{1-6}$- alkyl; an optionally substituted phenyl; or- N $(CH_3)_2$;
**$R_5$** and **$R_6$** are independently selected from a hydrogen; or $C_{1-6}$- alkyl **$R_7$** is selected from a hydrogen; $C_{1-6}$- alkyl; or a- $COR_8$
**$R_8$** is a $C_{1-6}$- alkyl;
**X** is selected from $NR_7$ or O;
**A** is a phenyl optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl; or an heteroaromatic ring optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl;
**n** is 0, 1 or 2,

and the salts, solvates and prodrugs thereof.

Also included within the scope of the invention are the isomers, polymorphs, isotopes, salts, solvates and prodrugs of the compounds of formula (I). Any reference to a compound of formula (I) throughout the present specification includes a reference to any isomer, polymorph, isotope, salt, solvate or prodrug of such compound of formula I.

The compounds of formula I may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula I, including all polymorphic forms ("polymorphs") thereof, are included within the scope of the invention.

Some of the compounds of the present invention may exist as several optical isomers and/or several diastereoisomers. Diastereoisomers can be separated by conventional techniques such as chromatography or fractional crystallization. Optical isomers can be resolved by conventional techniques of optical resolution to give optically pure isomers. This resolution can be carried out on any chiral synthetic intermediate or on the products of formula I. Optically pure isomers can also be individually obtained using enantiospecific synthesis. The present invention covers all individual isomers as well as mixtures thereof (for example racemic mixtures or mixtures of diastereomers), whether obtained by synthesis or by physically mixing them.

In addition, any formula given herein is intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, chlorine, and iodine, such as $^{2}H$, $^{3}H$, $^{11}C$, $^{13}C$, $^{14}C$, $^{15}N$, $^{18}O$, $^{17}O$, $^{31}P$, $^{32}P$, $^{36}S$, $^{18}F$, $^{36}Cl$, and $^{125}I$, respectively, Such isotopically labelled compounds are useful in metabolic studies (preferably with 14C), reaction kinetic studies (with, for example $^{2}H$ or $^{3}H$), detection or imaging techniques [such as positron emission tomography (PET) or single- photon emission computed tomography (SPECT) ] including drug or substrate tissue distribution assays, or in radioactive treatment of patients. In particular, an $^{18}F$ or $^{11}C$ labeled compound may be particularly preferred for PET or SPECT studies. Further, substitution with heavier isotopes such as deuterium (i.e., $^{2}H$) may afford certain therapeutic advantages resulting from greater metabolic stability, for example increased in vivo half- life or reduced dosage requirements. In addition to the unlabeled form, all isotopically labeled forms of the compounds of formula I are included within the scope of the invention.

"Halogen" or "halo" as referred in the present invention represent fluorine, chlorine, bromine or iodine.

The term "alkyl, " alone or in combination, means an acyclic radical, linear or branched, preferably containing from 1 to about 6 carbon atoms. Examples of such radicals include methyl, ethyl, n- propyl, isopropyl, n- butyl, isobutyl, sec- butyl, tert- butyl, pentyl, iso- amyl, hexyl, heptyl, octyl, and the like. Where no specific substitution is specified, alkyl radicals may be optionally substituted with groups consisting of hydroxy, sulfhydryl, methoxy, ethoxy, amino, cyano, chloro, and fluoro. The carbon atom content of various hydrocarbon- containing moieties is indicated by suffix designating a lower and upper number of carbon atoms in the moiety. Thus, for example, '$C_{1-6}$- alkyl' refers to alkyl of 1 to 6 carbon atoms, inclusive.

An "alkylene" linking group preferably contains 1- 4 carbon atoms and represents for example methylene, ethylene, propylene, butylene. The carbon atom content of various hydrocarbon- containing moieties is indicated by suffix designating a lower and upper number of carbon atoms in the moiety. Thus, for example, '$C_{1-4}$- alkylene' refers to an alkylene of 1 to 4 carbon atoms, inclusive.

An "alkenylene" linking group preferably contains 2 to 4 carbon atoms and represents for example ethenylene, 1, 3- propenylene, 1, 4- but- 1- enylene, 1, 4- but- 2- ethylene. The carbon atom content of various hydrocarbon- containing moieties is indicated by suffix designating a lower and upper number of carbon atoms in the moiety. Thus, for example, '$C_{2-4}$- alkenylene' refers to alkenylene of 2 to 4 carbon atoms, inclusive.

"Cycloalkyl" is preferably a monocyclic cycloalkyl containing from three to six carbon atoms. Examples include cyclopropyl, cyclobutyl, cyclopentyl, and cyclohexyl. The carbon atom content of various hydrocarbon- containing moieties is indicated by suffix designating a lower and upper number of carbon atoms in the moiety. Thus, for example, '$C_{3-6}$- cycloalkyl' refers to cycloalkyl of 3 to 6 carbon atoms, inclusive.

The term "carbocyclic", "carbocyclic ring" and "carbocyclyl" refer to a saturated, unsaturated or aromatic mono- or multi-ring cycloalkyl only formed from carbon atoms.

The terms "heterocycle", "heterocyclic ring" and "heterocyclyl" refer to a saturated, unsaturated or aromatic mono- or multi-ring cycloalkyl wherein one or more carbon atoms is replaced by N, S, or O. The terms "heterocycle", "heterocyclic ring system," and "heterocyclyl" include fully saturated ring structures such as piperazinyl, dioxanyl, tetrahydrofuranyl, oxiranyl, aziridinyl, morpholinyl, pyrrolidinyl, piperidinyl, thiazolidinyl, and others. The terms "heterocycle", "heterocyclic ring system," and "heterocyclyl" also include partially unsaturated ring structures such as dihydrofuranyl, dihydropyrrolyl, pyrazolinyl, imidazolinyl, pyrrolinyl, chromanyl, dihydrothienyl, and others. The term "heterocycle", "heterocyclic ring system," and "heterocyclyl" also include aromatic structures such as pyridyl, pyrimidinyl, pyridazinyl, pyrazinyl, thienyl, furanyl, pyrrolyl, pyrazolyl, imidazolyl, oxazolyl, isoxazolyl, thiazolyl, isothiazolyl, triazolyl, oxadiazolyl, thiadiazolyl, and tetrazolyl, optionally substituted.

The term "heteroaromatic ring" refers to an aromatic heterocyclic ring. Examples of "heteroaromatic ring" include pyridyl, pyrimidinyl, pyridazinyl, pyrazinyl, thionyl, furanyl, pyrrolyl, pyrazolyl, imidazolyl, oxazolyl, isoxazolyl, thiazolyl, isothiazolyl, triazolyl, oxadiazolyl, thiadiazolyl, and tetrazolyl, optionally substituted.

The term "ring" or "ring system" according to the present invention refers to ring systems comprising saturated, unsaturated or aromatic carbocyclic ring systems which contain optionally at least one heteroatom as ring member and which are optionally at least mono-substituted. Said ring systems may be condensed to other carbocyclic ring systems.

The term "monocyclic ring" refers to a ring system composed of a single ring.

The term "polycyclic ring" refers to a ring system composed of at least two rings.

The term "salt" must be understood as any form of an active compound used in accordance with this invention in which the said compound is in ionic form or is charged and coupled to a counter-ion (a cation or anion) or is in solution. This definition also includes quaternary ammonium salts and complexes of the active molecule with other molecules and ions, particularly complexes formed via ionic interactions. The definition particularly includes physiologically acceptable salts. This term must be understood as equivalent to "pharmaceutically acceptable salts".

The term "pharmaceutically acceptable salts" in the context of this invention means any salt that is tolerated physiologically (normally meaning that it is not toxic, particularly as a result of the counter- ion) when used in an appropriate manner for a treatment, particularly applied or used in humans and/or mammals. These pharmaceutically acceptable salts may be formed with cations or bases and, in the context of this invention, are understood to be salts formed by at least one compound used in accordance with the invention- normally an acid (deprotonated)- such as an anion and at least one physiologically tolerated cation, preferably inorganic, particularly when used on humans and/or mammals. Salts with alkali and alkali earth metals are particularly preferred, as well as those formed with ammonium cations ($NH_4^+$) . Preferred salts are those formed with (mono) or (di) sodium, (mono) or (di) potassium, magnesium or calcium. These physiologically acceptable salts may also be formed with anions or acids and, in the context of this invention, are understood as being salts formed by at least one compound used in accordance with the invention- normally protonated, for example in nitrogen- such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids- particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "solvate" in accordance with this invention should be understood as meaning any form of the active compound in accordance with the invention in which said compound is bonded by a non-covalent bond to another molecule (normally a polar solvent), especially including hydrates and alcoholates, for example methanolate.

The term "prodrug" is used in its broadest sense and encompasses those derivatives that are converted in vivo to the compounds of the invention. Examples of prodrugs include, but are not limited to, derivatives and metabolites of the compounds of formula (I) that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001 , Wiley) and "Design and Applications of Prodrugs" (H. Bundgaard ed., 1985, Harwood Academic Publishers).

The terms "prevention", "preventing", "preventive" "prevent" and "prophylaxis" refer to the capacity of a therapeutic to avoid, minimize or difficult the onset or development of a disease or condition before its onset.

The terms "treating" or "treatment" is meant at least a suppression or an amelioration of the symptoms associated with the condition afflicting the subject, where suppression and amelioration are used in a broad sense to refer to at least a reduction in the magnitude of a parameter, e.g., symptom associated with the condition being treated, such as pain. As such, the method of the present invention also includes situations where the condition is completely inhibited, terminated, such that the subject no longer experiences the condition.

A particular embodiment of the invention compounds of formula (I) are represented by general formula (Ia) :

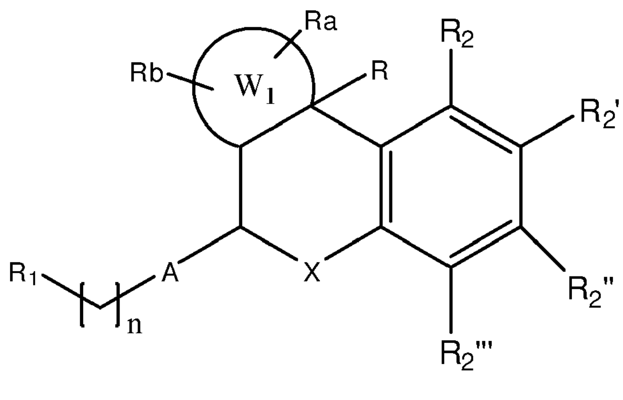

**(Ia)**

wherein **W1, X, A, R, Ra, Rb, $R_1$** and **n** have the same meaning as for compounds of general formula (I) ;
**$R_2$, $R_2$', $R_2$"** and **$R_2$"'** are independently selected from hydrogen; halogen; $C_{1-6}$ alkyl; $C_{1-6}$- haloalkyl;- O- $C_{1-6}$- alkyl;- O- $C_{1-6}$- haloalkyl; $C_{3-6}$cycloalkyl;- O- $C_{3-6}$cycloalkyl;- $NR_5SO_2R_6$; phenyl;- $CONH_2$ or- CN;
with the proviso that when W1 is a 6- membered saturated heterocyclic ring containing one oxygen as heteroatom atom and $R_1$ is a- COOH, at least one of **$R_2$, $R_{2'}$, $R_2$"** and **$R_2$"'** is different from H; and
with the proviso that when W1 is a 6- membered saturated heterocyclic ring containing one oxygen as heteroatom, $R_1$ is a- COOH and **$R_{2'}$** is methyl, **$R_{2''}$** must be different from methyl or that when W1 is a 6- membered saturated heterocyclic ring containing one oxygen as heteroatom, $R_1$ is a- COOH and **$R_2$"**
is methyl **$R_{2'}$** must be different from methyl; and
with the proviso that when W1 is a 6- membered saturated heterocyclic ring containing one oxygen as heteroatom, $R_1$ is a- COOH and **$R_{2'}$** is a methoxy group, a methyl group, a tertbutyl group, a chlorine or fluorine or **$R_2$"'** is methyl or chlorine, at least A must be substituted; and

In another particular and preferred embodiment of the invention the compounds of formula (I) and more particularly those with formula (Ia) are represented by the more particular general formula (Ia'):

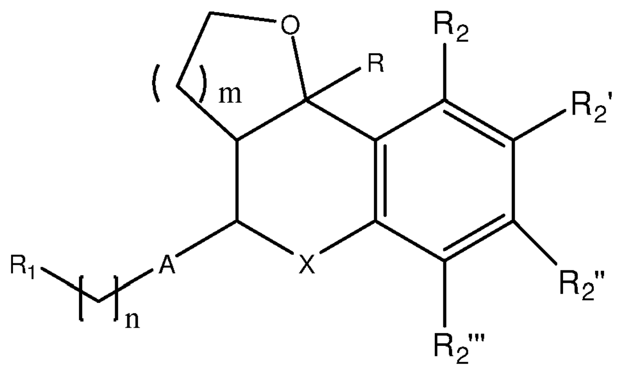

**(Ia')**

wherein **X, A, R, $R_1$, $R_2$, $R_2$', $R_2$"** and **$R_2$"'** and n have the same meaning as for compounds of formula (Ia); **m** is 1 or 2;
with the proviso that when **m**=2 and $R_1$ is a -COOH, at least one of **$R_2$, $R_2$', $R_2$"**
and **$R_2$"'** is different from H; and

with the proviso that when **m**=2, $R_1$ is a -COOH and **$R_2$'** is methyl, **$R_2$"** must be different from methyl or that when **m**=2, $R_1$ is a -COOH and **$R_2$"** is methyl **$R_2$'** must be different from methyl; and
with the proviso that when **m**=2, $R_1$ is a -COOH and **$R_2$'** is a methoxy group, a methyl group, a terbutyl group, a chlorine or fluorine or **$R_2$"'** is methyl or chlorine, at least A must be substituted.

In another particular embodiment of the invention compounds of general formula (I) are represented by compounds of general formula (Ib):

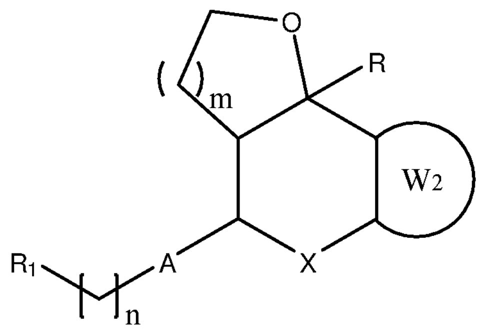

**(Ib)**

wherein **X, A, R, $R_1$** and n have the same meaning as for formula I; **m** is 1 or 2; and **W2** is a 5 or 6-membered heterocyclic ring containing 1 or 2 heteroatoms selected from N, O or S, optionally substituted by one or more **$R_2$** where **$R_2$** has the same meaning as for general formula (I).

Yet another embodiment of the invention comprises compound of general formula (I) represented by general formula (Ic)

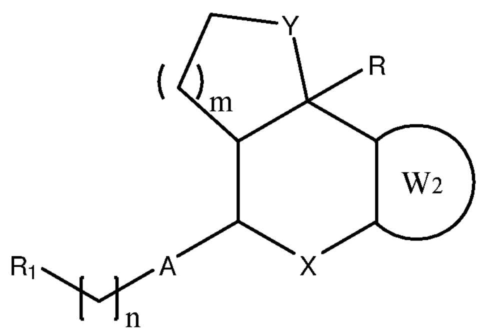

**(Ic)**

wherein **W2, X, A, R, $R_1$** and n have the same meaning as for general formula (I) ; **m** is 1 or 2 an **Y** is S or $NR_9$ where $R_9$ is hydrogen or $C_{1-6}$- alkyl.

In another particular embodiment of the invention compounds of general formula (I) are represented by compounds of general formula (Id):

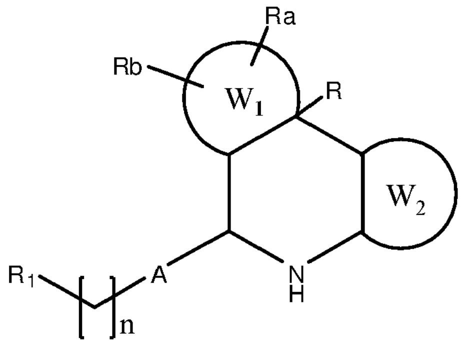

**(Id)**

wherein **W1, W2, A, R, Ra, Rb, $R_1$** and n have the same meaning as for compounds of general formula (I).

In another particular embodiment of the invention compounds of general formula (I) are represented by compounds of general formula (Ie):

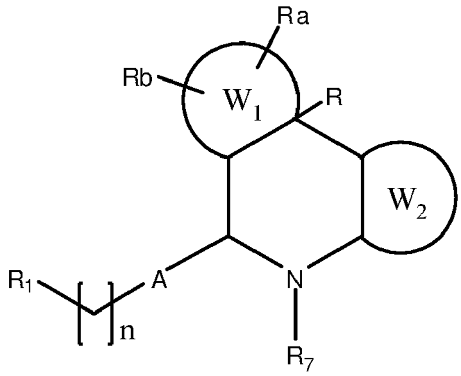

**(Ie)**

wherein **W1, W2, A, R, Ra, Rb, $R_1$, $R_7$** and n have the same meaning as for compounds of general formula (I).

In another particular embodiment of the invention compounds of general formula (I) are represented by compounds of general formula (If):

wherein **W1, W2, A, R, Ra, Rb, $R_1$, $R_8$** and n have the same meaning as for compounds of general formula (I).

In a preferred embodiment of the invention **$R_1$** is a -COOH group in the compounds of general formula (I), (Ia), (Ia'), (Ib) and (Ic).

In another preferred embodiment of the invention **A** is a phenyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted a $C_{1-6}$- alkyl; a pyridinyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted a $C_{1-6}$- alkyl; a pyrazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted a $C_{1-6}$- alkyl; an imidazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a $C_{3-6}$cycloalkyl optionally substituted a $C_{1-6}$- alkyl; or a thiazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a $C_{3-6}$cycloalkyl optionally substituted a $C_{1-6}$- alkyl.

In another preferred embodiment in the compounds of the invention **X** is $NR_7$ where $R_7$ is selected from a hydrogen or $C_{1-6}$- alkyl.

Another preferred embodiment is represented by compounds where **W2** is a benzene ring optionally substituted by one or more **$R_2$** where **$R_2$** has the same meaning as for general formula (I).

In another preferred embodiment in the compounds of the invention n is 0.

Among all the compounds encompassed by the general formula (I), (Ia), (Ia'), (Ib) and/or (Ic) the following compounds are particularly preferred:

- 4- ((4aR, 5S, 10bR)- 9- bromo- 3, 4, 4a, 5, 6, 10b- hexahydro- 2H- pyrano [3, 2- c] quinolin- 5- yl) benzoic acid;
- 4- ((4aR, 5R, 10bR)- 9- bromo- 3, 4, 4a, 5, 6, 10b- hexahydro- 2H- pyrano [3, 2- c] quinolin- 5- yl) benzoic acid;
- 4- ((4aR, 5S, 10bR)- 3, 4, 4a, 5, 6, 10b- hexahydro- 9- methyl- 2H- pyrano [3, 2- c] quinolin- 5- yl) benzoic acid;
- 4- ((4aR, 5R, 10bR)- 3, 4, 4a, 5, 6, 10b- hexahydro- 9- methyl- 2H- pyrano [3, 2- c] quinolin- 5- yl) benzoic acid;
- 4- ((4aR, 5R, 10bR)- 3, 4, 4a, 5, 6, 10b- hexahydro- 9- methoxy- 2H- pyrano [3, 2- c] quinolin- 5- yl)- 3- methylbenzoic acid;
- 4- ((4aR, 5R, 10bR)- 3, 4, 4a, 5, 6, 10b- hexahydro- 9- methyl- 2H- pyrano [3, 2- c] quinolin- 5- yl)- 3- methylbenzoic acid;
- 4- ((4aR, 5R, 10bR)- 9- chloro- 3, 4, 4a, 5, 6, 10b- hexahydro- 2H- pyrano [3, 2- c] quinolin- 5- yl)- 3- methylbenzoic acid;
- 4- ((4aR, 5S, 10bR)- 3, 4, 4a, 5, 6, 10b- hexahydro- 9- methoxy- 2H- pyrano [3, 2- c] quinolin- 5- yl)- 3- methylbenzoic acid;
- 4- ((4aR, 5S, 10bR)- 3, 4, 4a, 5, 6, 10b- hexahydro- 9- methoxy- 2H- pyrano [3, 2- c] quinolin- 5- yl)- 3- methylbenzoic acid (enantiomer 1) ;
- 4- ((4aR, 5S, 10bR)- 3, 4, 4a, 5, 6, 10b- hexahydro- 9- methoxy- 2H- pyrano [3, 2- c] quinolin- 5- yl)- 3- methylbenzoic acid (enantiomer 2) ;
- 4- ((4aR, 5S, 10bR)- 3, 4, 4a, 5, 6, 10b- hexahydro- 9- methyl- 2H- pyrano [3, 2- c] quinolin- 5- yl)- 3- methylbenzoic acid;
- 4- ((4aR, 5S, 10bR)- 3, 4, 4a, 5, 6, 10b- hexahydro- 9- methyl- 2H- pyrano [3, 2- c] quinolin- 5- yl)- 3- methylbenzoic acid (enantiomer 1) ;
- 4- ((4aR, 5S, 10bR)- 3, 4, 4a, 5, 6, 10b- hexahydro- 9- methyl- 2H- pyrano [3, 2- c] quinolin- 5- yl)- 3- methylbenzoic acid (enantiomer 2) ;
- 4- ((4aR, 5S, 10bR)- 9- chloro- 3, 4, 4a, 5, 6, 10b- hexahydro- 2H- pyrano [3, 2- c] quinolin- 5- yl)- 3- methylbenzoic acid;
- 4- ((4aR, 5S, 10bR)- 9- chloro- 3, 4, 4a, 5, 6, 10b- hexahydro- 2H- pyrano [3, 2- c] quinolin- 5- yl)- 3- methylbenzoic acid (enantiomer 1) ;
- 4- ((4aR, 5S, 10bR)- 9- chloro- 3, 4, 4a, 5, 6, 10b- hexahydro- 2H- pyrano [3, 2- c] quinolin- 5- yl)- 3- methylbenzoic acid (enantiomer 2) ;
- 4- (8- bromo- 2, 3, 3a, 4, 5, 9b- hexahydrofuro [3, 2- c] quinolin- 4- yl) benzoic acid;

In another embodiment, the sodium salt of the previous compounds is preferred.

In another aspect the invention refers to a process for preparing the compounds of the invention.

The compounds of the invention may be made by a variety of methods, including standard chemistry. Any previously defined variable will continue to have the previously defined meaning unless otherwise indicated. Illustrative general synthetic methods are set out below and then the preparation of specific compounds of the invention is described in more detail in the Experimental Section.

Compounds of general formula (I) may be obtained following the synthetic scheme depicted in scheme 1:

## Scheme 1

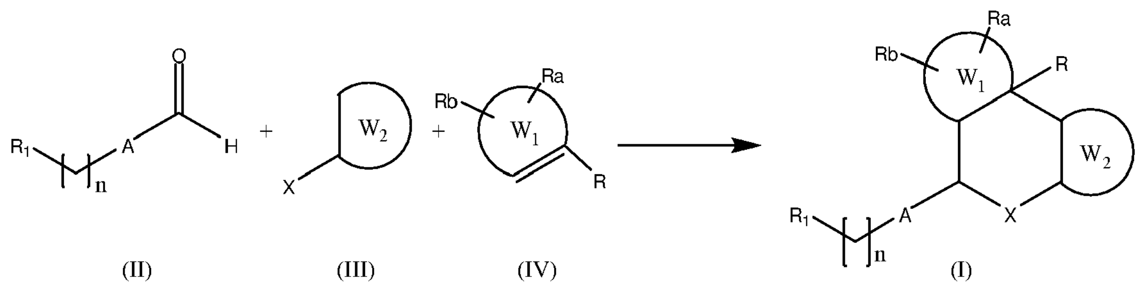

(II) (III) (IV) (I)

The reaction of an aldehyde of general formula (II) with an amine of general formula (III) in the presence of an alkene of general formula IV under acidic catalysis yield compounds of general formula I. Several Lewis acids such as scandium triflate ytterbium triflate, dysprosium triflate, copper (II) triflate, tin triflate, zinc triflate, zirconium triflate, borontrifluoroetherate, indium chloride, iron (III) chloride, ytterbium chloride, aluminium chloride, lanthanum (III) chloride, gadolinium (III) chloride, Samarium (III) chloride, titanium (II) chloride, titanium (IV) chloride, or organic acids such as trifluoroacetic acid trifluoromethane sulphonic acid, octane sulphonic acid, mixture of acetic acid with sulphuric acid can be used to induce this Povarov reaction. The reaction is carried out in the presence of an organic solvent such as acetonitrile, dichloromethane, 1, 2- dichloroethane, toluene, chloroform, hexane, tetrahydrofurane, diethyl ether at a temperature between room temperature and the solvent boiling point.

Alternatively, the reaction can be performed in two steps as shown below in scheme 2.

**Scheme 2**

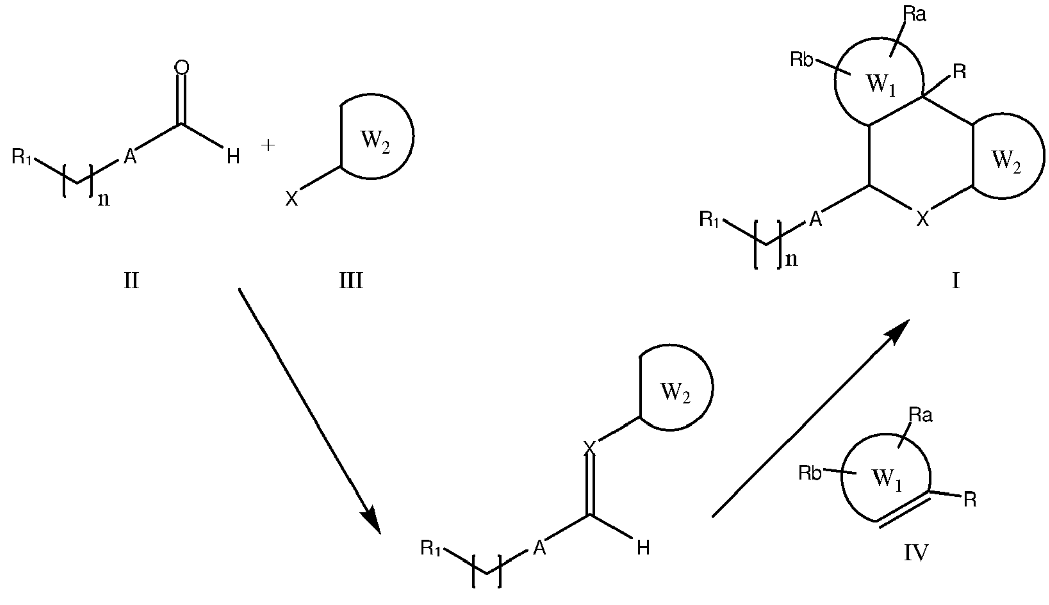

II III I IV V

In an initial step, reaction of aldehydes of formula II with amines of formula III in the presence of a dehydrating agent such as molecular sieves trimethyl orthoformate, acetic acid, titanium (IV) chloride, anhydrous magnesium sulphate or dehydration by Dean Stark system using p-toluenesulfonic acid in a solvent such as chloroform dichloromethane, toluene, methanol at a temperature between 20 and 150°C to yield the corresponding imines V that upon isolation are allowed to further react with the corresponding alkenes of general formula IV in the presence of the same acids, solvents and conditions depicted above.

The reactions of scheme 1 and 2 are only applicable to the preparation of compounds of general formula I where X is $NR_7$.

Compounds of general formula II, 111 and IV are either commercially available or are synthesized according to methods known in the art.

In the particular case in which X is $NR_7$ with $R_7$ being $C_{1-6}$- alkyl, compounds of general formula (Ie) can be obtained from compounds of general formula (Id) by reaction with an alkylating agent such as VI wherein Y is an halogen atom such as chlorine, bromine or iodide or a sulphonate ester such as mesilate, tosilate or trifluoromethanesulphonate in basic media such as sodium hydride and in a solvent such as THF or DMF at a temperature from 0 to 150°C (see scheme 3) . Alternativelly, compounds of general formula (Ie) can be obtained by reductive amination of aldehydes of general formual VII with amines of general formula (Id) in acidic media such as acetic acid and in a protic solvent such as methanol or ethanol and with a reductive agent such as sodium borohydride, sodium triacetoxyborohydride or sodium cyanoborohydride at a temperature from 0°C to the boiling point of the solvent. As an alternative to the acidic media a Lewis acid such as zinc chloride can be used.

**Scheme 3**

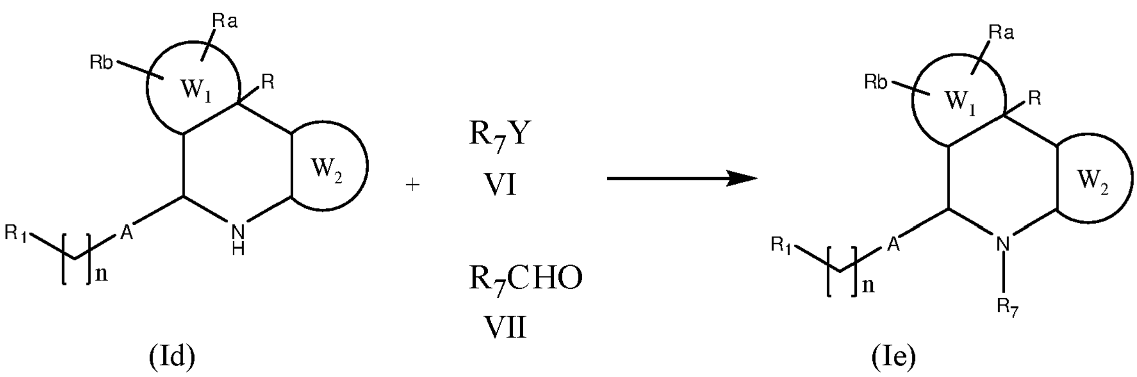


(Id) (Ie)

In the particular case in which X is $NR_7$ with $R_7$ being a $-COR_8$, compounds of general formual (If) (see scheme 4 below), can be obtained from compounds of general formula (Id) by reaction of an acylating agent such as VIII in which R8 is a $C_{1-6}$-alkyl and Y is an halogen. The reaction takes place in basic media such as triethylamine, pyridine or diisopropylethylamine with a solvent such as dichloromethane, DMF, THF or pyridine at a temperature between 20 and 150°C and in a standard reactor or in a microwave apparatus.

## Scheme 4

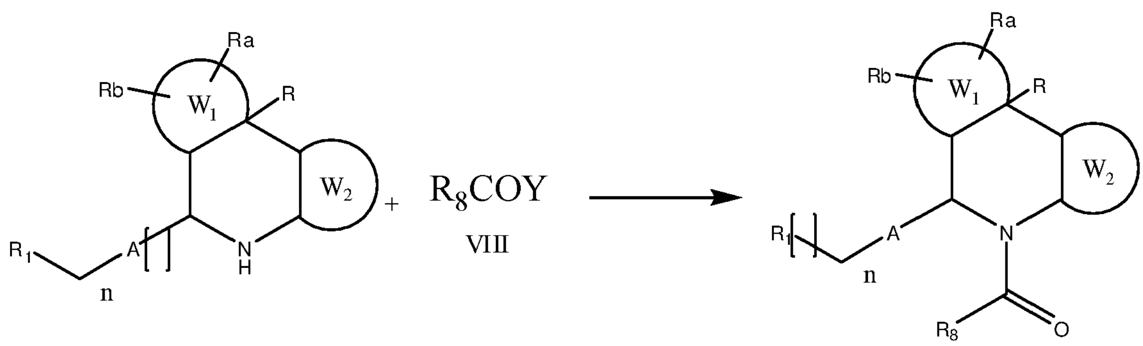

(Id)

(If)

When the defined groups R1 to R8 are susceptible to chemical reaction under the conditions of the hereinbefore described processes or are incompatible with said processes, conventional protecting groups may be used in accordance with standard practice, for example see T.W. Greene and P.G.M. Wuts in "protective Groups in Organic Chemistry", 3rd Edition, John Wiley&Sons (1999). It may be that deprotection will form the last step in the synthesis of compounds of formula (I).

An additional aspect of the invention relates to the therapeutic use of the compounds of general formula (I). As mentioned above, compounds of general formula (I) show a strong affinity to EP1 receptors. For this reason, they are suitable for the treatment and/or the prophylaxis of disorders and diseases mediated by EP1 receptors.

Compounds of the invention are particularly useful for modulating pain. The compounds of the present invention can treat or prevent the pain associated with several pathological conditions comprising, among others, inflammatory related pain (Hall et al. 2007) including low back and neck pain, skeletal pain, post- partum pain, toothache, sprains and straits, myositis, neuralgia, synovitis, arthritis, including rheumatoid arthritis, degenerative joint diseases (osteoarthritis), gout and ankylosing spondylitis, bursitis, burns including radiation and corrosive chemical injuries and sunburns; postoperative pain (Omote et al. 2001) including dental procedures; neuropathic pain (Kawahara et al. 2001) ; visceral pain (Sarkar et al. 2003) ; tension headache; cluster headaches; migraine and the like.

Moreover, by inhibition of prostanoid- induced smooth muscle contraction by antagonizing contractile prostanoids or mimicking relaxing prostanoids, EP1 modulators may be used in the treatment of motility- related disorders (with or without pain) such as gastroinstestinal disorders (Sarkar et al. 2003; Mizuguchi et al 2010) and urinary incontinence and other urinary tract diseases (Teramura et al. 2000; Lee et al. 2007; Okada et al., 2010; Wilbraham et al 2010; Miki et al 2010), dysmenorrhea and preterm labour.

The compounds of the invention can also be useful in prostaglandin-mediated proliferation disorders such as in diabetic retinopathy and tumour angiogenesis, cancer (Watanabe et al. 1999; Niho et al. 2005), the inhibition of cellular neoplasic transformations and metastatic tumour growth.

They can further be used in the treatment of neurodegenerative diseases (including senile dementia, Alzheimer's disease, Pick's disease, Huntington's chorea, Parkinson's disease, Creutzfeldt-Jakob disease, or Amyotrophic Lateral Sclerosis) (Li et al. 2011), neuroprotection/stroke (Abe et al 2009), glaucoma (Woodward et al 1997), bone loss (osteoporosis) and the proportion of bone formation (treatment of fractures) (Zhang et al 2011; Lee et al. 2007) and other bone diseases such as Paget's disease.

As PGE2-induced hyperthermia in the rat is mediated predominantly through the EP1 receptor (Hönemann et al. 2001; Oka et al. 2003) different kinds of fever as rheumatic fever, symptoms associated with influenza or other viral infections as well as common cold can be also target diseases for EP1 modulators.

The compounds of the invention can also have a cytoprotective activity in patients under different gastrointestinal disorders as related with chemotherapy, or irritable bowel disease. Other diseases that can be treated or prevented with the compounds of the invention include gastrointestinal bleeding, coagulation disorders including anaemia such as hypoprothrombinemia, haemophilia or other bleeding problems; kidney diseases (nephritis (Rahal et al. 2006), particularly

mesangial proliferative glomerulonephritis and nephritic syndrome); thrombosis, and occlusive vascular diseases.

In this sense, compounds of formula (I) are suitable to treat or to prevent diseases or disorders comprising inflammatory related pain including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; common cold, gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases.

The invention thus relates to a compound of formula (I) for use in the treatment and/or prophylaxis of an EP1-mediated disease or disorder. In one embodiment, the EP1-mediated disease or disorder is selected from the group consisting of pain, motility-related disorders, gastrointestinal disorders, urinary tract diseases, cancer, neurodegenerative diseases, stroke, glaucoma, bone diseases, fever, coagulation disorders and occlusive vascular diseases. In a preferred embodiment, the EP1-mediated disease or disorder is pain. In another embodiment, the EP1-mediated disease or disorder is selected from the group consisting of inflammatory related pain including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; common cold, gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases. In a preferred embodiment, the EP1-mediated disease or disorder is pain comprising inflammatory related pain, including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine.

A related aspect refers to the use of at least one compound of general formula (I) for the manufacture of a medicament for the treatment and/or prophylaxis diseases or disorders mediated by EP1 receptors or in which EP1 receptors are involved.

In one embodiment, the EP1-mediated disease or disorder is selected from the group consisting of pain, motility-related disorders, gastrointestinal disorders, urinary tract diseases, cancer, neurodegenerative diseases, stroke, glaucoma, bone diseases, fever, coagulation disorders and occlusive vascular diseases. In a preferred embodiment, the EP1-mediated disease or disorder is pain. In another embodiment, the EP1-mediated disease or disorder is selected from the group consisting of inflammatory related pain including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hypertermia including different types of fever as rheumatic fever; symptoms associated with influenza or other viral infections; common cold, gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases. In another embodiment, the

EP1-mediated disease or disorder is selected from the group consisting of inflammatory related pain (including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns); postoperative pain; neuropathic pain; visceral pain; tension headache; cluster headaches; and migraine.

An aspect of the invention related to the therapeutic use of the compounds of general formula (I) is a method of treatment and/or prophylaxis of disorders and diseases mediated by EP1 receptors which comprises administering to a patient in need thereof a therapeutically effective amount of at least one compound of general formula (I). In one embodiment, the EP1-mediated disease or disorder is selected from the group consisting of pain, motility-related disorders, gastrointestinal disorders, urinary tract diseases, cancer, neurodegenerative diseases, stroke, glaucoma, bone diseases, fever, coagulation disorders and occlusive vascular diseases. In a preferred embodiment, the EP1-mediated disease or disorder is pain. In another embodiment, the EP1-mediated disease or disorder is selected from the group consisting of inflammatory related pain including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; common cold, gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases. In another embodiment, the EP1-mediated disease or disorder is selected from the group consisting of inflammatory related pain (including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns); postoperative pain; neuropathic pain; visceral pain; tension headache; cluster headaches; and migraine.

The amount of active ingredient that must be administered to the patient depends on the patient's weight, the type of application, the condition and severity of the disease. Normally, in human beings 1 to 1500 mg of the active compound is administered daily in one or several doses.

A further aspect of the invention regards a pharmaceutical composition which comprises a compound of general formula (I), and at least a pharmaceutically acceptable carrier, additive, adjuvant or vehicle.

The auxiliary materials or additives can be selected among carriers, excipients, support materials, lubricants, fillers, solvents, diluents, colorants, flavour conditioners such as sugars, antioxidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The pharmaceutical composition in accordance with the invention can be adapted to any form of administration, be it orally or parenterally, for example pulmonarily, nasally, rectally and/or intravenously. Therefore, the formulation in accordance with the invention may be adapted for topical or systemic application, particularly for dermal, subcutaneous, intramuscular, intra-articular, intraperitoneal, pulmonary, buccal, sublingual, nasal, percutaneous, vaginal, oral or parenteral application.

Suitable preparations for oral applications are tablets, pills, chewing gums, capsules, granules, drops or syrups.Suitable preparations for parenteral applications are solutions, suspensions, reconstitutable dry preparations or sprays.

The compounds of the invention are formulated as deposits in dissolved form or in patches, for percutaneous application.

Skin applications include ointments, gels, creams, lotions, suspensions or emulsions.

The preferred form of rectal application is by means of suppositories.

## EXPERIMENTAL SECTION

The following abbreviations are used along the experimental section:

DMF: dimethylformamide

EtAcO: ethyl acetate

EtOH: ethanol

IPA: Isopropyl alcohol

HCl: Hydrochloric acid

DCM: dichloromethane

NMR: nuclear magnetic resonance

MeOH: methanol

THF: tetrahydrofurane

rt: room temperature

ACN: Acetonitrile

TFA: Trifluoroacetic acid

i-PrMgCl: isopropylmagnesium chloride

$Sc(OTf)_3$: Scandium trifluoromethanesulfonate

LiOH: Lithium hydroxide

The following HPLC methods for LC-MS spectra have been used:

Method 1: X-Bridge C18, 3.5 μm 4.6 x 50 mm column; temperature: 25 °C; rate 2.0 mL/min; eluent: A = HCOOH 1 mM, B = ACN:MeOH:HCOOH (1:1:0.0005); gradient: 100% to 5% A in 12 min, 5% A1 min, 5 to 100% A1 min.

Method 2: X-Bridge C18, 3.5 μm 4.6 x 50 mm column; temperature: 25 °C; rate 2.0 mL/min; eluent: A = HCOOH 1 mM, B = ACN:MeOH:HCOOH (1:1:0.0005); gradient: 95 to 0% A in 5.5 min, 0% A 3 min, 0 to 95% A 0.5 min.

Method 3: X-Bridge C18, 3.5 μm 4.6 x 50 mm column; temperature: 25 °C; rate 2.0 mL/min; eluent: A = HCOOH 1 mM, B = ACN:MeOH:HCOOH (1:1:0.0005); gradient: 95 to 0% A in 4.5 min, 0% A 1.5 min, 0 to 95% A 0.5 min.

Separation of enantiomers was carried out in a Preparative Waters HPLC (pump Waters 600; Manual injector Waters 2700; Detector Waters 2487; Software: MassLynx 3.5). Using preparative packed column with Chiralpak IA (20 μm) using a self-packing system (195 mm x 50 mm). Flux 55 mL/min and fraction collection λ = 254 nm.

Optical purity of the separated enantiomers were determinated by HPLC (Chiralpak IA 250 x 4.6 mm, 5 μm, 10 μL, 1 mL/min). Using the same eluents of the preparative chiral chromatography.

Optical rotation of the separated enantiomers were determined in a polarimeter JASCO P-2000 with a cell of 1 mL.

<u>**Intermediate compounds**</u>

***Intermediate compound 1: methyl 4-formyl-3-methylbenzoate***

A solution of methyl 4- iodo- 3- methylbenzoate (21.7 mmol, 6 g) in dry THF (120 mL) was purged with argon. The solution was cooled to- 15°C, *i*- PrMgCl (2M in THF, 108.5 mmol, 54.25 mL), and DMF (130.2 mmol, 10 mL) were added and the reaction was stirred for 2h at- 15°C and then warmed to 25°C and allowed to react for an additional hour. The reaction was quenched with aqueous 1N HCl and extracted with EtAcO. The organic layer was washed with brine, dried over anhydrous sodium sulfate, filtered and concentrated. The resulting crude was purified by flash chromatography on silica gel using an elution of 6% ethylacetate in hexanes to give methyl 4- formyl- 3- methylbenzoate (3.08 g. Yield: 80%) .

$^1H$ NMR (400 MHz, $CDCl_3$) δ 10.35 (1H, s), 8.00 (1H, d, *J*=8Hz), 7.99 (1H, s), 7.86 (1H, d, *J*=8Hz), 3.95 (3H, s), 2.72 (3H, s)

LC- MS: $t_R$ = 2.85 $[M+H]^+$ = not ion. (method 3)

***Intermediate compound 2 and 3: methyl 4-((4aR, 5R, 10bR)-3, 4, 4a, 5, 6, 10b-hexahydro-9-methoxy-2H pyrano [3,2-c]quinolin-5-yl)-3-methylbenzoate (2) and methyl 4-((4aR, 5S, 10bR)-3, 4, 4a, 5, 6, 10b-hexahydro-9-methoxy-2H-pyrano[3,2-c]quinolin-5-yl)-3-methylbenzoate (3). Separation of (+/-)-methyl 4-((4aR, 5S, 10bR)-3, 4, 4a, 5, 6, 10b-hexahydro-9-methoxy-2H pyrano[3,2-c]quinolin-5-yl)-3-methylbenzoate (3)***

Method 1: To a solution of methyl 4- formyl- 3- methylbenzoate (intermediate compound **1**) (1.92 mmol, 342 mg) in anhydrous ACN (20 mL) was added p- anisidine (1.92 mmol, 236 mg), 3, 4- dihydro- 2H- pyran (3.84 mmol, 0.35 mL) and Sc $(OTf)_3$ (0.096 mmol, 48 mg) . The reaction mixture was stirred 16 h at room temperature. Solvent was evaporated and purification of the crude material (4: 6 mixture of exo/ endo regioisomers) by flash chromatography on silica gel using an elution of 18% ethylacetate in hexanes afforded the exo- isomer **3** methyl 4- ((4a*R*, 5S, 10b*R*)- 3, 4, 4a, 5, 6, 10b- hexahydro- 9- methoxy- 2*H*- pyrano [3, 2- c] quinolin- 5- yl)- 3- methylbenzoate (154 mg. Yield: 22%) and the endo- isomer **2** methyl 4- ((4a*R*, 5*R*, 10b*R*)- 3, 4, 4a, 5, 6, 10b- hexahydro- 9- methoxy- 2*H*- pyrano [3, 2- c] quinolin- 5- yl)- 3- methylbenzoate (310 mg. Yield: 43%) .

Method 2: In order to increase the reaction ratio of exo- isomer to a solution of methyl 4- formyl- 3- methylbenzoate (intermediate compound **1**) (5.61 mmol, 1 g) in anhydrous ACN (60 mL) p- anisidine (5.1 mmol, 629 mg), 3, 4- dihydro- 2H- pyran (10.2 mmol, 0.93 mL) and TFA (1.12 mmol, 0.087mL) were added. The reaction mixture was stirred 16h at room temperature. Solvent was evaporated and purification the crude material (4: 2 mixture of exo/ endo regioisomers) by flash chromatography on silica gel using an elution of 25% ethylacetate in hexanes afforded the exo- isomer **3** methyl 4- ((4a*R*, 5*S*, 10b*R*)- 3, 4, 4a, 5, 6, 10b- hexahydro- 9- methoxy- 2*H*- pyrano [3, 2- c] quinolin- 5- yl)- 3- methylbenzoate (914 mg. Yield: 45%) and the endo- isomer **2** methyl 4- ((4a*R*, 5*R*, 10b*R*)- 3, 4, 4a, 5, 6, 10b- hexahydro- 9- methoxy- 2*H*- pyrano [3, 2- *c*] quinolin- 5- yl)- 3- methylbenzoate (227 mg. Yield: 11%) .

***Intermediate compound 2:*** *methyl 4-((4aR, 5R, 10bR)-3, 4, 4a, 5, 6, 10b-hexahydro-9-methoxy-2H-pyrano[3,2-c]quinolin-5-yl)-3-methylbenzoate (endo-isomer)*

$^1H$ NMR (400 MHz, DMSO- $d_6$) δ 7.81 (1 H, d, *J*= 8Hz), 7.79 (1 H, s), 7.69 (1 H, d, *J*=8Hz), 6.79 (1 H, s), 6.65 (2H, m), 5.52 (1 H, s), 5.24 (1 H, 5.6Hz), 4.77 (1 H, s), 3.85 (3H, s), 3.66 (3H, s), 3.49 (1 H, d, *J*=12Hz), 3.27- 3.25 (1 H, m), 2.38 (3H, s), 2.02 (1 H, m), 1.43- 1.39 (3H, m), 1.04 (1 H, m) .

LC- MS: $t_R$ = 4.07 $[M+H]^+$ = 368 (method 2)

***Intermediate compound 3:*** *methyl 4-((4aR, 5S, 10bR)-3, 4, 4a, 5, 6, 10b-hexahydro-9-methoxy-2H-pyrano[3,2-c]quinolin-5-yl)-3-methylbenzoate (exo-isomer)*

$^1H$ NMR (400 MHz, $CDCl_3$) δ 7.87- 7.84 (2H, m), 7.49 (1H, d, *J*=8Hz), 6.85 (1 H, d, *J*=2.8Hz), 6.75 (1H, dd, *J*=2.8 & 8.4Hz), 6.50 (1H, d, *J*=8.8Hz), 4.91 (1H, d, *J*=10Hz), 4.42 (1H, d, *J*=3.2Hz), 4.03 (1H, m), 3.91 (3H, s), 3.77 (3H, s), 3.72- 7.67 (1H, m), 2.50 (3H, s), 2.26- 2.22 (1H, m), 1.71- 1.69 (2H, m), 1.46- 1.39 (2H, m) .

LC- MS: $t_R$ = 3.87 $[M+H]^+$ = 368 (method 2)

Separation of 448 mg of (+/- )- methyl 4- ((4a*R*, 5*S*, 10b*R*)- 3, 4, 4a, 5, 6, 10b- hexahydro- 9- methoxy- 2*H*- pyrano [3, 2- c] quinolin- 5- yl)- 3- methylbenzoate (**3**) by preparative chiral chromatography was carried out using heptane/IPA (95: 5) as eluents to yield:

143 mg of the enantiomer 1:

Preparative HPLC: $t_R$ = 42.00 min

Optical Purity: 99.9% ee

158 mg of the enantiomer 2:

Preparative HPLC: $t_R$ =65 min

Optical Purity: 99.9% ee

***Intermediate compound 4 and 5: methyl 4-((4aR, 5R, 10bR)-3, 4, 4a, 5, 6, 10b-hexahydro-9-methyl-2H-pyrano [3,2-c]quinolin-5-yl)-3-methylbenzoate (4) and methyl 4-((4aR, 5S, 10bR)-3, 4, 4a, 5, 6, 10b-hexahydro-9-methyl-2H pyrano[3,2-c]quinolin-5-yl)-3-methylbenzoate (5)***

The following compounds were prepared using the same methodology as in Intermediate compound 2 and 3 method 1, using *p*- toluidine (1.97 mmol, 211 mg) . Solvent was evaporated and purification of the crude material (1: 1.75 mixture of exo/ endo regioisomers) by flash chromatography on silica gel using an elution of 7% ethylacetate in hexanes afforded the exo- isomer **5** methyl 4- ((4a*R*, 5*S*, 10b*R*)- 3, 4, 4a, 5, 6, 10b- hexahydro- 9- methyl- 2*H*- pyrano [3, 2- c] quinolin- 5- yl)- 3- methylbenzoate (136 mg. Yield: 20%) and the endo- isomer **4** methyl 4- ((4a*R*, 5*R*, 10b*R*)- 3, 4, 4a, 5, 6, 10b- hexahydro- 9- methyl- 2*H*- pyrano [3, 2- *c*] quinolin- 5- yl)- 3- methylbenzoate (363mg.Yield: 52%)

The following compounds were also prepared using the same methodology as in Intermediate compound 2 and 3 method 2, using p-toluidine (5.1 mmol, 547 mg). Solvent was evaporated and purification of the crude material (6:4 mixture of exo/endo regioisomers) by flash chromatography on silica gel using an elution of 15% ethylacetate in hexanes afforded the exo-isomer **5** (1g. Yield: 50%) and the endo-isomer **4** (290 mg. Yield: 14%).

***Intermediate compound 4: methyl 4-((4aR, 5R, 10bR)-3, 4, 4a, 5, 6, 10b-****hexahydro-9-methyl-2H-pyrano[3,2-c]quinolin-5-yl)-3-methylbenzoate (endo-isomer)*

$^1$H NMR (400 MHz, DMSO- $d_6$) δ 7.83- 7.80 (2H, m), 7.67 (1H, d, *J*=8Hz), 7.02 (1H, s), 6.81 (1H, d, *J*=8Hz), 6.60 (1H, d, *J*=8Hz), 5.67 (1H, s), 5.23 (1H, d, *J*=5.2Hz), 4.80 (1 H, d, *J*=1.6Hz), 3.85 (3H, s), 3.49- 3.46 (1 H, m), 3.26- 3.20 (1 H, m), 2.38 (3H, s), 2.18 (3H, s), 2.03- 2.01 (1 H, m), 1.50- 1.46 (1 H, m), 1.44- 1.38 (2H, m), 1.04- 1.03 (1 H, m)

LC- MS: $t_R$ = 4.50 $[M+H]^+$ = 352 (method 2)

***Intermediate compound 5:*** *methyl 4-((4aR, 5S, 10bR)-3, 4, 4a, 5, 6, 10b-hexahydro-9-methyl-2H-pyrano[3,2-c]quinolin-5-yl)-3-methylbenzoate (exo-isomer)*

LC- MS: $t_R$ = 4.33 $[M+H]^+$ = 352 (method 2)

***Intermediate compound 6 and 7: methyl 4-((4aR, 5R, 10bR)-9-chloro-3, 4, 4a, 5, 6, 10b-hexahydro-2H-pyrano [3,2-c]quinolin-5-yl)-3-methylbenzoate (6) and methyl 4-((4aR, 5S, 10bR)-9-chloro-3, 4, 4a, 5, 6, 10b-hexahydro-2H pyrano[3,2-c]quinolin-5-yl)-3-methylbenzoate (7)***

The following compounds were prepared using the same methodology as in Intermediate compound 2 and 3 method 1 using 4- chloroaniline (1.97 mmol, 252 mg) . Solvent was evaporated and purification of the crude material (1: 1 mixture of exo/ endo regioisomers) by flash chromatography on silica gel using an elution of 6% ethylacetate in hexanes afforded the exo- isomer **7** methyl 4- ((4a*R*, 5*S*, 10b*R*)- 9- chloro- 3, 4, 4a, 5, 6, 10b- hexahydro- 2*H*- pyrano [3, 2- *c*] quinolin- 5- yl)- 3- methylbenzoate (226 mg. Yield: 31%) and the endo- isomer **6** methyl 4- ((4a*R*, 5*R*, 10b*R*)- 9- chloro- 3, 4, 4a, 5, 6, 10b- hexahydro- 2*H*- pyrano [3, 2- *c*] quinolin- 5- yl)- 3- methylbenzoate (277 mg. Yield: 37%)

***Intermediate compound 6:*** *methyl 4-((4aR, 5R, 10bR)-9-chloro-3, 4, 4a, 5, 6, 10b-hexahydro-2H-pyrano[3,2-c]quinolin-5-yl)-3-methylbenzoate (endo-isomer)*

$^1$H NMR (400 MHz, $CDCl_3$) δ 7.90- 7.88 (2H, m), 7.64 (1 H, d, *J*=8Hz), 7.41 (1 H, d, *J*=2.4Hz), 7.04 (1 H, dd, *J*= 2.4 & 8.4Hz), 6.55 (1 H, d, *J*=8.8Hz), 5.26 (1 H, d, *J*=5.6Hz), 4.87 (1 H, d, *J*=2.4Hz), 3.918 (3H, s), 3.63- 3.59 (1 H, m), 3.44- 3.41 (1 H, m), 2.38 (3H, s), 2.18- 2.15 (1 H, m), 1.89- 1.81 (1 H, m), 1.60- 1.48 (3H, m), 1.28- 1.24 (1H, m) .

LC- MS: $t_R$ = 4.63 $[M+H]^+$ = 372 (method 2)

***Intermediate compound 7:*** *methyl 4-((4aR, 5S, 10bR)-9-chloro-3, 4, 4a, 5, 6, 10b-hexahydro-2H-pyrano[3,2-c]quinolin-5-yl)-3-methylbenzoate (exo-isomer)*

$^1$H NMR (400 MHz, $CDCl_3$) δ 7.88- 7.85 (2H, m), 7.50- 7.44 (1 H, m), 7.24 (1 H, d, *J*=2.4Hz), 7.04 (1 H, dd, *J*=2.4 & 8.4Hz), 6.47 (1 H, d, *J*=8.8Hz), 4.93 (1 H, d, *J*=9.6Hz), 4.41 (1 H, d, *J*=3.2 Hz), 4.05- 4.01 (1 H, m), 3.91 (3H, s), 3.69- 3.67 (1 H, m), 2.49 (3H, s), 2.25- 2.21 (1 H, m), 1.72- 1.68 (1 H, m), 1.58- 1.49 (2H, m), 1.47- 1.44 (1 H, m) .

LC- MS: $t_R$ = 4.48 $[M+H]^+$ = 372 (method 2)

The following examples illustrate the scope of the invention.

**Examples of compounds of general formula I**

***Example 1 and 2: 4-((4aR,5S,10bR)-9-bromo-3,4,4a,5,6,10b-hexahydro-2H pyrano[3,2-c]quinolin-5-yl)benzoic acid (1) and 4-((4aR,5R,10bR)-9-bromo-3,4,4a,5,6,10b-hexahydro-2H-pyrano[3,2-c]quinolin-5-yl)benzoic acid (2)***

The following compound was prepared using the same methodology as in Intermediate compound 2 and 3 method 1, using 4- formylbenzoic acid (150 mg, 1 mmol), 4- aminobromobenzene (1 mmol, 172 mg) . Solvent was evaporated and purification of the crude material (55: 44 mixture of exo/ endo regioisomers) by flash chromatography on silica gel using an elution of 6% methanol in ethylacetates afforded a 51: 48 mixture of exo/ endo isomers as a white solid (92 mg, 23%) which was purified using reverse $C_{18}$ chromatography using water (0.1% formic acid) and acetonitrile (0.1% formic acid) as eluents. Only pure fractions were collected to give 11 mg of 4- ((4a*R*, 5*S*, 10b*R*)- 9- bromo- 3, 4, 4a, 5, 6, 10b- hexahydro- 2*H*- pyrano [3, 2- c] quinolin- 5- yl) benzoic acid **1** and 14 mg of 4- ((4a*R*, 5*R*, 10b*R*)- 9- bromo- 3, 4, 4a, 5, 6, 10b- hexahydro- 2*H*- pyrano [3, 2- c] quinolin- 5- yl) benzoic acid **2** both as a white solids.

**Example 1:** *4-((4aR,5S, 10bR)-9-bromo-3,4,4a,5,6, 10b-hexahydro-2H-pyrano[3,2-c]quinolin-5-yl)benzoic (exo-isomer):*

$^1$H NMR (400 MHz, DMSO- $d_6$) δ 7.93 (2H, d, *J* = 8 Hz), 7.51 (2H, d, *J* = 8 Hz), 7.17 (1 H, d, *J* = 2.4 Hz), 7.11 (1 H, dd, *J* = 8.8 & 2.4 Hz), 6.55 (1 H, d, *J* = 8.5 Hz), 6.45 (1 H, s), 4.56 (1 H, d, *J* = 10 Hz), 4.27 (1 H, d, *J* = 2.4 Hz), 3.85 (1 H, d, *J* = 10.8 Hz), 3.58 (1 H, td, *J* = 10.8 & 2.4 Hz), 1.93- 1.91 (1 H, m), 1.78- 1.69 (1 H, m), 1.65- 1.57 (1 H, m), 1.30- 1.26 (1 H, m), 1.23- 1.18 (1 H, m) .

LC- MS: $t_R$ = 7.25 $[M+H]^+$= 388/390 (method 1) .

**Example 2:** *4-((4aR,5R, 10bR)-9-bromo-3,4,4a,5,6,10b-hexahydro-2H-pyrano[3,2-c]quinolin-5-yl)benzoic acid (endo-isomer):*

$^1$H NMR (400 MHz, DMSO- $d_6$) δ 7.93 (2H, d, *J* = 8.4 Hz), 7.51 (2H, d, *J* = 8 Hz), 7.23 (1 H, d, *J* = 1.6 Hz), 7.12 (1 H, td, *J* = 8.4, 2.4 & 0.4 Hz), 6.65 (1 H, d, *J* =8.8 Hz), 6.27 (1 H, s), 5.22 (1 H, d, *J* = 5.6 Hz), 4.70 (1 H, d, *J* = 2.4 Hz), 3.50 (1 H, d, *J* = 11.2Hz), 3.17- 3.28 (1H, m), 2.06- 2.04 (1H, m), 1.37- 1.31 (2H, m), 1.30- 1.20 (1 H, m), 1.03- 1.00 (1 H, m) .

LC- MS: $t_R$ = 7.47 $[M+H]^+$= 388/390 (method 1) .

***Example 3 and 4: 4-((4aR,5S,10bR)-3,4,4a,5,6,10b-hexahydro-9-methyl-2H pyrano[3,2-c]quinolin-5-yl)benzoic acid (3) and 4-((4aR,5R,10bR)-3,4,4a,5,6,10b-hexahydro-9-methyl-2H-pyrano[3,2-c]quinolin-5-yl)benzoic acid (4)***

The following compounds were prepared using the same methodology as in Intermediate compound 2 and 3 method 1, using p-toluidine (1.6 mmol, 178 mg). Solvent was evaporated and purification of the crude material (29:51 mixture of exo/endo regioisomers) by flash chromatography on silica gel using an elution of 55% EtAcO in hexanes afforded a 33:61 mixture of exo/endo isomers as a white solid (170 mg, 32%) which was purified using reverse $C_{18}$ chromatography using water (0.1% formic acid) and acetonitrile (0.1% formic acid) as eluents to give 14 mg of exo-isomer **3** and 50 mg of endo-isomer **4** both as a white solids. (Overall yield= 12%).

**Example 3:** *4-((4aR,5S, 10bR)-3,4,4a,5,6,10b-hexahydro-9-methyl-2H-pyrano[3,2-c]quinolin-5-yl)benzoic acid (exo-isomer)*

$^1$H NMR (400 MHz, DMSO- $d_6$) δ 7.98 (2H, d, *J* = 8 Hz), 7.58 (2H, d, *J* = 8.4 Hz), 6.92 (1H, s), 6.86 (1 H, d, *J* = 8.4 Hz), 6.55 (1 H, d, *J* = 8.4 Hz), 6.04 (1 H, s), 4.60 (1H, d, *J* = 10 Hz), 4.29 (1 H, d, *J* = 2.4 Hz), ), 3.92 (1 H, d, *J* = 10.8 Hz), 3.61- 3.66 (1H, m), 2.19 (3H, s), 1.98- 1.94 (1 H, m), 1.84- 1.74 (1 H, m), 1.70- 1.62 (1 H, m), 1.35- 1.26 (2H, m)

LC- MS: $t_R$ = 6.60 min $[M+H]^+$= 324 (method 1) .

**Example 4:** *4-((4aR,5R, 10bR)-3,4,4a,5,6,10b-hexahydro-9-methyl-2H-pyrano[3,2-c]quinolin-5-yl)benzoic acid (endo-isomer)*

$^1$H NMR (400 MHz, DMSO- $d_6$) δ 7.94 (2H, d, *J* = 8.4 Hz), 7.54 (2H, d, *J* = 8.4 Hz), 7.01 (1 H, s), 6.81 (1H, d, *J* = 8 Hz), 6.60 (1H, d, *J* = 8 Hz), 5.82 (1 H, s), 5.21 (1 H, d, *J* = 5.2 Hz), 4.66 (1 H, d, *J* = 1.6 Hz), ), 3.47 (1 H, d, *J* = 11.2 Hz), 3.33- 3.20 (1 H, m), 2.18 (3H, s), 2.07- 2.02 (1 H, m), 1.37- 1.32 (3H, m), 1.04- 1.00 (1 H, m) .

LC- MS: $t_R$ = 6.88 min $[M+H]^+$= 324 (method 1) .

***Example 5: 4-((4aR, 5R, 10bR)-3, 4, 4a, 5, 6, 10b-hexahydro-9-methoxy-2H pyrano[3,2-c]quinolin-5-yl)-3-methylbenzoic acid***

To a solution of 4- ((4a*R*, 5*R*, 10b*R*)- 3, 4, 4a, 5, 6, 10b- hexahydro- 9- methoxy- 2*H* pyrano [3, 2- c] quinolin-5- yl)- 3- methylbenzoate (**intermediate compound 2**, endo- isomer) (0.84 mmol, 310 mg) in THF (10 mL) was added 1 M LiOH (1.68 mmol, 1.7 mL) . The reaction mixture was stirred 16 h at room temperature. More 1 M LiOH (1.68 mmol, 1.7 mL) was added and it was stirred for 48 h at room temperature. Solvent was evaporated and purification of the crude material by reverse $C_{18}$ chromatography using water (0.1% formic acid) and acetonitrile (0.1% formic acid) as eluents afforded the 4- ((4a*R*, 5*R*, 10b*R*)- 3, 4, 4a, 5, 6, 10b- hexahydro- 9- methoxy- 2*H*- pyrano [3, 2- c] quinolin- 5- yl)- 3-methylbenzoic acid. (99mg. Yield: 33%) .

$^1$H NMR (400 MHz, DMSO- $d_6$) $\delta$ 7.79- 7.76 (2H, m), 7.63 (1H, d, *J*=8Hz), 6.79 (1 H, s), 6.65 (2H, m), 5.50 (1 H, s), 5.23 (1 H, d, *J*=5.6Hz), 4.76 (1 H, s), 3.66 (3H, s), 3.49 (1 H, d, J=11.2Hz), 3.28- 3.25 (1 H, m), 2.36 (3H, s), 2.07-1.99 (1 H, m), 1.41- 1.39 (3H, m), 1.20- 1.15 (1H, m) .

LC- MS: $t_R$ = 6.33 $[M+H]^+$ = 354 (method 1) .

*Example 6: 4-((4aR, 5R, 10bR)-3, 4, 4a, 5, 6, 10b-hexahydro-9-methyl-2H pyrano[3,2-c]quinolin-5-yl)-3-methylbenzoic acid*

The following compound was prepared using the same methodology as in example 5 using methyl 4- ((4aR, 5R, 10bR)- 3, 4, 4a, 5, 6, 10b- hexahydro- 9- methyl- 2H- pyrano [3, 2- c] quinolin- 5- yl)- 3- methylbenzoate (intermediate compound 4) (1.03 mmol, 363 mg) . More 1 M LiOH (2 mmol, 2 mL) was added and it was stirred for 24h at room temperature. Solvent was evaporated and purification of the crude material by reverse $C_{18}$ chromatography using water (0.1% formic acid) and acetonitrile (0.1% formic acid) as eluents afforded the 4- ((4aR, 5R, 10bR)- 3, 4, 4a, 5, 6, 10b-hexahydro- 9- methyl- 2*H*- pyrano [3, 2- c] quinolin- 5- yl)- 3- methylbenzoic acid (267 mg. Yield: 77%) .

$^1$H NMR (400 MHz, DMSO- $d_6$) $\delta$ 7.79- 7.77 (2H, m), 7.63 (1H, d, J=8Hz), 7.02 (1 H, s), 6.80 (1 H, dd, J=1.6 & 8Hz), 6.60 (1 H, d, J=8Hz), 5.66 (1 H, s), 5.23 (1 H, d, J=5.6Hz), 4.79 (1 H, d, J=1.6Hz), 3.47 (1 H, d, J= 10.8Hz), 3.30- 3.27 (1 H, m), 2.37 (3H, s), 2.18 (3H, s), 2.05- 2.01 (1 H, m), 1.41- 1.38 (3H, m), 1.06- 1.04 (1 H, m) .

LC- MS: $t_R$ = 7.22 $[M+H]^+$ = 338 (method 1) .

***Example 7: 4-((4aR, 5R, 10bR)-9-chloro-3, 4, 4a, 5, 6, 10b-hexahydro-2H pyrano[3,2-c]quinolin-5-yl)-3-methylbenzoic acid***

The following compound was prepared using the same methodology as in example 5 using methyl 4- ((4a*R*, 5*R*, 10b*R*)- 9- chloro- 3, 4, 4a, 5, 6, 10b- hexahydro- 2*H*- pyrano [3, 2- c] quinolin- 5- yl)- 3- methylbenzoate (**intermediate compound 6**) (0.75 mmol, 277 mg) . More 1 M LiOH (1.5 mmol, 1.5 mL) was added and it was stirred for 24 h at room temperature. Solvent was evaporated and purification of the crude material by reverse $C_{18}$ chromatography using water (0.1% formic acid) and acetonitrile (0.1% formic acid) as eluents afforded the 4- ((4a*R*, 5*R*, 10b*R*)- 9- chloro- 3, 4, 4a, 5, 6, 10b- hexahydro- 2*H*- pyrano [3, 2- c] quinolin- 5- yl)- 3- methylbenzoic acid (120 mg. Yield: 45%) .

$^1$H NMR (400 MHz, DMSO- $d_6$) $\delta$ 7.08- 7.78 (2H, m), 7.60 (1H, d, *J*=8Hz), 7.13 (1 H, d, *J*=1.6Hz), 7.02 (1 H, dd, *J*=2 & 8.4Hz), 6.71 (1 H, d, *J*=8.4Hz), 6.12 (1 H, s), 5.25 (1H, d, *J*=5.6Hz), 4.86 (1 H, d, *J*=1.2Hz), 3.52 (1 H, d, *J*=11.2Hz), 3.25- 3.20 (1H, m), 2.37 (3H, s), 2.07- 2.03 (1 H, m), 1.41- 1.33 (3H, m), 1.08- 1.06 (1 H, m) .

LC- MS: $t_R$ = 7.57 $[M+H]^+$ = 358 (method 1) .

***Example 8: 4-((4aR, 5S, 10bR)-3, 4, 4a, 5, 6, 10b-hexahydro-9-methoxy-2H pyrano[3,2-c]quinolin-5-yl)-3-methylbenzoic acid***

The following compound was prepared using the same methodology as in example 5 using (+/- )- methyl 4- ((4a*R*, 5*S*, 10b*R*)- 3, 4, 4a, 5, 6, 10b- hexahydro- 9- methoxy- 2*H*- pyrano [3, 2- c] quinolin- 5- yl)- 3- methylbenzoate (**intermediate compound 3**) (0.42 mmol, 154 mg) . More 1 M LiOH (0.84 mmol, 0.8 mL) was added and it was stirred for 24 h at room temperature. Solvent was evaporated and purification of the crude material by reverse $C_{18}$ chromatography using water (0.1 % formic acid) and acetonitrile (0.1 % formic acid) as eluents afforded the (+/- ) 4- ((4a*R*, 5*S*, 10b*R*)- 3, 4, 4a, 5, 6, 10b- hexahydro- 9- methoxy- 2*H*- pyrano [3, 2- c] quinolin- 5- yl)- 3- methylbenzoic acid. (55 mg. Yield: 37%) .

$^1$H NMR (400 MHz, DMSO- $d_6$) $\delta$ 7.78 (1 H, s), 7.75 (1 H, d, *J*=8Hz), 7.43 (1 H, d, *J*=8.4Hz), 6.69- 6.65 (2H, m), 6.52 (1 H, d, *J*=8.4Hz), 5.73 (1 H, s), 4.70 (1 H, d, *J*=9.2Hz), 4.32 (1 H, d, *J*=2Hz), 3.85- 3.82 (1 H, m), 3.65 (3H, s), 3.60- 3.55 (1 H, m), 2.46 (3H, s), 2.09- 2.07 (1 H, m), 1.66- 1.60 (2H, m), 1.36- 1.25 (2H, m) .

LC- MS: $t_R$ = 5.87 $[M+H]^+$ = 354 (method 1) .

***Enantiomer 1: 4-((4aR, 5S, 10bR)-3, 4, 4a, 5, 6, 10b-hexahydro-9-methoxy-2H-pyrano[3,2-c]quinolin-5-yl)-3-methylbenzoic acid (8a)***

The following compound was prepared using the same methodology as in example 5 using methyl 4- ((4a*R*, 5*S*, 10b*R*)- 3, 4, 4a, 5, 6, 10b- hexahydro- 9- methoxy- 2*H*- pyrano [3, 2- *c*] quinolin- 5- yl)- 3- methylbenzoate (**intermediate compound 3, enantiomer 1**) (0.39 mmol, 143 mg) and 1M LiOH (2.33 mmol, 2.3 mL) . The reaction mixture was stirred for 16 h at room temperature. Water was added and the mixture was cooled with an ice/ water bath and 1 N HCl was added until a white solid appeared. The mixture was stirred at 0- 5ºC for 30 min and the solid was separated by filtration, washed with cold water and dried under vacuum at 40°C to give 92 mg of the enantiomer 1 title compound (**8a**) . (Yield: 67%) .

***Enantiomer 2*: *4-((4aR, 5S, 10bR)-3, 4, 4a, 5, 6, 10b-hexahydro-9-methoxy-2H-pyrano[3,2-c]quinolin-5-yl)-3-methylbenzoic acid (8b)***

The following compound was prepared using the same methodology as in example 5 using methyl 4- ((4a*R*, 5*S*, 10b*R*)- 3, 4, 4a, 5, 6, 10b- hexahydro- 9- methoxy- 2*H*- pyrano [3, 2- c] quinolin- 5- yl)- 3- methylbenzoate (**intermediate compound 3, enantiomer 2**) (0.43 mmol, 158 mg) and 1M LiOH (2.60 mmol, 2.6 mL) . The reaction mixture was stirred for 16 h at room temperature. Water was added and the mixture was cooled with an ice/ water bath and 1 N HCl was added until a white solid appeared. The mixture was stirred at 0- 5ºC for 30 min and the solid was separated by filtration, washed with cold water and dried under vacuum at 40°C to give 103 mg of the enantiomer 2 title compound (**8b**) . (Yield: 68%) .

***Example 9*: *4-((4aR, 5S, 10bR)-3, 4, 4a, 5, 6, 10b-hexahydro-9-methyl-2H pyrano[3,2-c]quinolin-5-yl)-3-methylbenzoic acid. Separation of (+/-)-4-((4aR, 5S, 10bR)-3, 4, 4a, 5, 6, 10b-hexahydro-9-methyl-2H-pyrano[3,2-c]quinolin-5-yl)-3-methylbenzoic acid (9)***

The following compound was prepared using the same methodology as in example 5 using methyl 4- ((4a*R*, 5*S*, 10b*R*)- 3, 4, 4a, 5, 6, 10b- hexahydro- 9- methyl- 2*H*- pyrano [3, 2- c] quinolin- 5- yl)- 3- methylbenzoate (**intermediate compound 5**) (0.39 mmol, 136 mg) . More 1M LiOH (0.8 mmol, 0.8 mL) was added and it was stirred for 24 h at room temperature. Solvent was evaporated and purification of the crude material by reverse $C_{18}$ chromatography using water (0.1 % formic acid) and acetonitrile (0.1 % formic acid) as eluents afforded the (+/)- 4- ((4a*R*, 5*S*, 10b*R*)- 3, 4, 4a, 5, 6, 10b- hexahydro- 9- methyl- 2*H*- pyrano [3, 2- c] quinolin- 5- yl)- 3- methylbenzoic acid (62 mg. Yield: 47%) .

$^1$H NMR (400 MHz, DMSO- $d_6$) $\delta$ 7.78 (1 H, s), 7.74 (1 H, d, *J*=8Hz), 7.42 (1H, d, *J*=8Hz), 6.88 (1 H, s), 6.81 (1 H, dd, *J*=2 & 8Hz), 6.48 (1H, d, *J*= 8Hz), 5.88 (1H, s), 4.71 (1 H, d, *J*=9.6Hz), 4.29 (1 H, d, *J*=2.8Hz), 3.83- 3.81 (1 H, m), 3.57- 3.52 (1 H, m), 2.45 (3H, s), 2.15 (3H, s), 2.10- 2.07 (1 H, m), 1.69- 1.59 (2H, m), 1.42- 1.36 (1 H, m), 1.35- 1.24 (1 H, m) .

LC- MS: $t_R$ = 6.90 $[M+H]^+$ = 338 (method 1) .

Separation of 300 mg of (+/- )- 4- ((4a*R*, 5*S*, 10b*R*)- 3, 4, 4a, 5, 6, 10b- hexahydro- 9- methyl- 2*H*- pyrano [3, 2- *c*] quinolin- 5- yl)- 3- methylbenzoic acid (**9**) by preparative chiral chromatography was carried out using Heptane/ EtOH/TFA (95: 5: 0.1) as eluents to yield:

75 mg of the enantiomer 1 (**9a**):

Preparative HPLC: $t_R$ = 37.00 min

Optical Purity: 99.9% ee

90 mg of the enantiomer 2 (**9b**):

Preparative HPLC: $t_R$ =57 min

Optical Purity: 99.9% ee

***Example 10: 4-((4aR, 5S, 10bR)-9-chloro-3, 4, 4a, 5, 6, 10b-hexahydro-2H pyrano[3,2-c]quinolin-5-yl)-3-methylbenzoic acid. Separation of (+/-)-4-((4aR, 5S, 10bR)-9-chloro-3, 4, 4a, 5, 6, 10b-hexahydro-2H-pyrano[3,2-c]quinolin-5-yl)-3-methylbenzoic acid (10)***

The following compound was prepared using the same methodology as in example 5 using methyl 4- ((4a*R*,

5*S*, 10b*R*)- 9- chloro- 3, 4, 4a, 5, 6, 10b- hexahydro- 2*H*- pyrano [3, 2- c] quinolin- 5- yl)- 3- methylbenzoate (**intermediate compound 7**) (0.61 mmol, 226 mg) . More 1M LiOH (2.5 mmol, 2.5 mL) was added and it was stirred for 48 h at room temperature. Solvent was evaporated and purification of the crude material by reverse $C_{18}$ chromatography using water (0.1% formic acid) and methanol (0.1% formic acid) as eluents afforded the (+/- )- 4- ((4a*R*, 5*S*, 10b*R*)- 9- chloro- 3, 4, 4a, 5, 6, 10b- hexahydro- 2*H*- pyrano [3, 2- *c*] quinolin- 5- yl)- 3- methylbenzoic acid (104 mg. Yield: 48%) .

$^1$H NMR (400 MHz, DMSO- $d_6$) δ 7.79 (1H, s), 7.76 (1 H, d, *J*=8.4Hz), 7.42 (1 H, d, *J*=8Hz), 7.08 (1 H, d, *J*=2.8Hz), 7.01 (1H, dd, *J*=2.8 & 8.8Hz), 6.58 (1 H, d, *J*=8.8 Hz), 6.34 (1 H, s), 4.73 (1 H, d, *J*=9.2Hz), 4.33 (1H, d, *J*= 3.2Hz), 3.83- 3.80 (1 H, m), 3.06- 3.56 (1 H, m), 2.45 (3H, s), 2.10- 2.07 (1H, m), 1.68- 1.62 (2H, m), 1.38- 1.35 (1 H, m), 1.27- 1.23 (1 H, m) .

LC- MS: $t_R$ = 7.30 $[M+H]^+$ = 358 (method 1) .

Separation of 366 mg of (+/- )- 4- ((4a*R*, 5*S*, 10b*R*)- 9- chloro- 3, 4, 4a, 5, 6, 10b- hexahydro- 2*H*- pyrano [3, 2- c] quinolin- 5- yl)- 3- methylbenzoic acid (**10**) by preparative chiral chromatography was carried out using Heptane/IPA (95: 5) as eluents to yield:

80 mg of the enantiomer 1 (**10a**):

Preparative HPLC: $t_R$ = 71.00 min

Optical Purity: 99.9% ee

86 mg of the enantiomer 2 (**10b**):

Preparative HPLC: $t_R$ = 96 min

Optical Purity: 99.9% ee

***Example 11*: *4-(8-bromo-2,3,3a,4,5,9b-hexahydrofuro[3,2-c]quinolin-4-yl)benzoic acid***

A catalytic amount of scandium trifluoromethanesulfonate (0.05 eq., 0.05 mmol, 24 mg) in anhydrous acetonitrile (5 mL) was added to a mixture of 4-formylbenzoic acid (150 mg, 1 mmol), 4-aminobromobenzene (1 mmol, 172 mg), and 2,3-dihydrofurane (2 eq., 2 mmol, 0.15 mL). The reaction mixture was stirred at room temperature for 16 h. Solvent was evaporated and purification of the crude material (42:58 mixture of exo/endo regioisomers) by flash chromatography on silica gel using an elution of 6% methanol in ethylacetate afforded the title compound as a 42:58 mixture of exo/endo regioisomers as a white solid (98 mg, Yield: 26%).

$^1$H NMR (400 MHz, DMSO- $d_6$) δ 7.93 (2H, d, *J* = 8 Hz), 7.56 (2H, d, *J* = 8 Hz), 7.30 (1 H, d, *J* = 2.4 Hz, exo), 7.21 (1 H, d, *J* = 2.4 Hz, endo), 7.16 (1H, dd, *J* = 8.4 & 2.4 Hz, exo), 7.11 (1 H, dd, *J* = 8.8 & 2.4 Hz, endo), 6.68 (1 H, d *J* = 8.8 Hz, endo), 6.66 (1 H, d *J* = 8.8 Hz, exo), 6.49 (1 H, s, exo), 6.22 (1 H, s, endo), 5.12 (1 H, d, *J* = 7.6 Hz, endo), 4.70 (1 H, d, *J* = 2.8 Hz, endo), 4.43 (1 H, d, *J*= 5.2 Hz, exo), 3.88 (1 H, q, *J* = 8.4 Hz, exo), 3.70 (1 H, d, *J* = 10.4 Hz, exo), 3.63- 3.69 (1 H, m), 3.62- 3.55 (1 H, m), 2.71- 2.65 (1 H, m), 2.25- 2.31 (1 H, m, exo), 1.96- 1.81 (1 H, m), 1.56- 1.50 (1 H, m, exo), 1.33- 1.25 (1 H, m)

LC- MS: $t_R$ = 6.73 $[M+H]^+$= 374/376 (method 1) .

**Examples of biological activity**

In the following examples the biological activity of compounds of formula (I) is described.

**Human EP1 receptor radioligand binding assay**

To investigate binding properties of EP1 receptor ligands to human EP1 receptor, transfected HEK- 293 cell membranes and [3H]- PGE2 (Perkin Elmer) were used. In 96- well plates the assay was carried out with a total reaction volume of 250 μl, containing 25 μl of membrane suspension (30 μg protein/ well), 25 μl of [3H]- PGE2 (10 nM) in either absence or presence of 25 μl of either buffer or PGE2 (10 μM) for total and non- specific binding, respectively. Binding buffer contained 10 mM MES, 1 mM MgCl2 and 1 mM EDTA at pH 6.0. Plates were incubated at 25 °C for 60 minutes. After the incubation period, 200 μl of incubate were transferred to MultiScreen HTS, FB plates (Millipore), filtered and plates were washed 6 times with ice- cold 10 mM MES, 1 mM MgCl2 and 1 mM EDTA at pH 6.0. Filters were dried and counted in a MicroBeta scintillation counter (Perkin- Elmer) using EcoScint liquid scintillation cocktail.

Percentage inhibition was calculated relating compounds activity to the 0% inhibition of the wells incubated with 10 nM [3H]-PGE2 alone (total binding) and 100% inhibition of the wells incubated with 10 nM [3H]-PGE2 plus 10

μM PGE2 (non-specific binding). Competition binding curves were carried out by assaying 6 different concentrations with duplicated points. The inhibition constant (Ki) of each compound was calculated by the Cheng-Prusoff equation:

$$K_i = IC_{50} / (1 + [L] / KD)$$

where $IC_{50}$ is the concentration of compound that displaces the binding of radioligand by 50%, [L] is the free concentration of radioligand and KD is the dissociation constant of each radioligand. $IC_{50}$ values were obtained by fitting the data with non-linear regression using XIFit software (IDBS).

The results obtained in the biological assays disclosed above with representative compounds of formula (I) are shown in the Table below in which A means a Ki < 100nM, B means a Ki value between 100nM and 500nM and C means a Ki > 500 nM.

| EXAMPLE | Ki binding |
|---|---|
| Example 2 | C |
| Example 5 | B |
| Example 6 | B |
| Example 10 | A |

**References**

Abe T, Kunz A, Shimamura M, Zhou P, Anrather J, Iadecola C. (2009) The neuroprotective effect of prostaglandin E2 EP1 receptor inhibition has a wide therapeutic window, is sustained in time and is not sexually dimorphic. J Cereb Blood Flow Metab. 29 (1) : 66- 72.

Asbóth G, Phaneuf S, Europe- Finner GN, Tóth M, Bernal AL. (1996) Prostaglandin E2 activates phospholipase C and elevates intracellular calcium in cultured myometrial cells: involvement of EP1 and EP3 receptor subtypes. Endocrinology. 137 (6) : 2572- 9.

Baba H, Kohno T, Moore KA, Woolf CJ. (2001) Direct activation of rat spinal dorsal horn neurons by prostaglandin E2 The Journal of Neuroscience, 21 (5) : 1750- 1756.

Breyer MD, Breyer RM. (2000) Prostaglandin receptors: their role in regulating renal function. Curr Opin Nephrol Hypertens. 2000 Jan; 9 (1) : 23- 9.

Candelario- Jalil E, Slawik H, Ridelis I, Waschbisch A, Akundi RS, Hüll M, Fiebich BL. (2005) Regional distribution of the prostaglandin E2 receptor EP1 in the rat brain: accumulation in Purkinje cells of the cerebellum. J Mol Neurosci. 27 (3) : 303- 10.

Coleman, R. A., Prostanoid Receptors. IUPHAR compendium of receptor characterization and classification, 2nd edition, 338-353, 2000.

Dirig DM, Yaksh TL. (1999) In vitro prostanoid release from spinal cord following peripheral inflammation: effects of substance P, NMDA and capsaicin. Br J Pharmacol. 126 (6) : 1333- 40.

Durrenberger PF, Facer P, Casula MA, Yiangou Y, Gray RA, Chessell IP, Day NC, Collins SD, Bingham S, Wilson AW, Elliot D, Birch R, Anand P. (2006) Prostanoid receptor EP1 and Cox-2 in injured human nerves and a rat model of nerve injury: a time-course study. BMC Neurol. 4;6:1.

Giblin GM, Bit RA, Brown SH, Chaignot HM, Chowdhury A, Chessell IP, Clayton NM, Coleman T, Hall A, Hammond B, Hurst DN, Michel AD, Naylor A, Novelli R, Scoccitti T, Spalding D, Tang SP, Wilson AW, Wilson R. (2007) The discovery of 6- [2- (5- chloro- 2- { [ (2, 4- difluorophenyl) methyl] oxy} phenyl)- 1- cyclopenten- 1- yl]- 2- pyridinecarboxylic acid, GW848687X, a potent and selective prostaglandin EP1 receptor antagonist for the treatment of inflammatory pain. Bioorg Med Chem Lett. 17 (2) : 385- 9.

Guay J., Bateman, K., Gordon R., Mancini J., Riendeau D. (2004) Carrageenan-induced paw edema in rat elicits a predominant prostaglandin E2 (PGE2) response in the central nervous system associated with the induction of microsomal PGE2 synthase-1 J. Biol Chem 2004. 279, 24866-24872.

Hall, A., Billinton A., Giblin G.M. (2007) EP1 antagonists for the treatment of inflammatory pain. Curr Opin. Drug Discov. Devel. 10 (2007) 597-612.

Hall A, Brown SH, Budd C, Clayton NM, Giblin GM, Goldsmith P, Hayhow TG, Hurst DN, Naylor A, Anthony Rawlings D, Scoccitti T, Wilson AW, Winchester WJ. (2009) Discovery of GSK345931 A: An EP (1) receptor antagonist with efficacy in preclinical models of inflammatory pain. Bioorg Med Chem Lett. 19 (2) : 497- 501.

Hönemann CW, Heyse TJ, Möllhoff T, Hahnenkamp K, Berning S, Hinder F, Linck B, Schmitz W, van Aken H. (2001) The inhibitory effect of bupivacaine on prostaglandin E (2) (EP (1) ) receptor functioning: mechanism of action. Anesth Analg. 93 (3) : 628- 634.

Johansson T, Narumiya S, Zeilhofer HU. (2011) Contribution of peripheral versus central EP1 prostaglandin receptors to inflammatory pain. Neurosci Lett. 495 (2) : 98- 101.

Kawahara H, Sakamoto A, Takeda S, Onodera H, Imaki J, Ogawa R. (2001) A prostaglandin E2 receptor subtype EP1 receptor antagonist (ONO- 8711) reduces hyperalgesia, allodynia, and c- fos gene expression in rats with chronic nerve constriction. Anesth Analg. 93 (4) : 1012- 7.

Lee T, Hedlund P, Newgreen D, Andersson KE. (2007) Urodynamic effects of a novel EP (1) receptor antagonist in normal rats and rats with bladder outlet obstruction. J Urol. 177 (4) : 1562- 1567.

Lee C.M, Genetos DC, You Z, Yellowley CE. (2007b) Hypoxia regulates PGE (2) release and EP1 receptor expression in osteoblastic cells. J Cell Physiol. 212 (1) : 182- 188

Li X, Cudaback E, Keene CD, Breyer RM, Montine TJ. (2011) Suppressed microglial E prostanoid receptor 1 signaling selectively reduces tumor necrosis factor alpha and interleukin 6 secretion from toll- like receptor 3 activation. Glia. 59 (4) : 569- 576

Lin CR, Amaya F, Barrett L, Wang H, Takada J, Samad TA, Woolf CJ (2006) Prostaglandin E2 receptor EP4 contributes to inflammatory pain hypersensitivity. J Pharmacol Exp Ther. 319 (3) : 1096- 103.

Ma W, Eisenach JC. (2003) Four PGE2 EP receptors are up- regulated in injured nerve following partial sciatic nerve ligation. Exp Neurol. 183 (2) : 581- 92.

Miki, T.; Matsunami, M.; Okada, H.; Matsuya, H.; Kawabata, A (2010) ONO-8130, an EP1 antagonist, strongly attenuates cystitis-related bladder pain caused by cyclophosphamide in mice. J Pharmacol Sci 112(Suppl. 1): Abst P1 J-1-2

Minami T, Uda R., Horiguchi S., Ito S. Hyodo M., Hayaishi O. (1994) Allodynia evoked by intrathecal administration fo prostaglandin E2 to conscious mice. Pain, 1994, 57: 217-223.

Minami T, Nakano H, Kobayashi T, Sugimoto Y, Ushikubi F, Ichikawa A, Narumiya S, Ito S. (2001) Characterization of EP receptor subtypes responsible for prostaglandin E2- induced pain responses by use of EP1 and EP3 receptor knockout mice. Br J Pharmacol. 133 (3) : 438- 44.

Mizuguchi S, Ohno T, Hattori Y, Ae T, Minamino T, Satoh T, Arai K, Saeki T, Hayashi I, Sugimoto Y, Narumiya S, Saigenji K, Majima M. (2010) Roles of prostaglandin E2- EP1 receptor signaling in regulation of gastric motor activity and emptying. Am J Physiol Gastrointest Liver Physiol. 299 (5) : G1078- 1086

Moriyama T, Higashi T, Togashi K, Iida T, Segi E, Sugimoto Y, Tominaga T, Narumiya S, Tominaga M. (2005) Sensitization of TRPV1 by EP1 and IP reveals peripheral nociceptive mechanism of prostaglandins. Mol Pain. 1: 3.

Nakayama Y, Omote K, Namiki A. (2002) Role of prostaglandin receptor EP1 in the spinal dorsal horn in carrageenan-induced inflammatory pain. Anesthesiology. 97 (5) : 1254- 62.

Nakayama Y, Omote K, Kawamata T, Namiki A. (2004) Role of prostaglandin receptor subtype EP1 in prostaglandin E2- induced nociceptive transmission in the rat spinal dorsal horn. Brain Res. 1010 (1- 2) : 62- 8.

Narumiya S., Sugimoto Y., Ushikubi F. (1999) Protanoid receptors: structures, properties, and functions. Physiol Rev. 79 (1999) 1193-1226.

Niho N, Mutoh M, Kitamura T, Takahashi M, Sato H, Yamamoto H, Maruyama T, Ohuchida S, Sugimura T, Wakabayashi K. (2005) Suppression of azoxymethane- induced colon cancer development in rats by a prostaglandin E receptor EP1- selective antagonist. Cancer Sci. 96 (5) : 260- 264.

Oidda H., Namba T., Sugimoto Y., Ushikubi F., Ohishi H., Ichikawa A. et al (1995) In situ hybridization studies of prostacyclin receptor mRNA expression in various mouse organs. Br J Pharmacol 1995, 116, 2828-2837.

Oka T, Oka K, Saper CB. (2003) Contrasting effects of E type prostaglandin (EP) receptor agonists on core body temperature in rats. Brain Res. 968 (2) : 256- 262.

Oka T, Hosoi M, Oka K, Hori T. (1997) Biphasic alteration in the trigeminal nociceptive neuronal responses after intracerebroventricular injection of prostaglandin E2 in rats. Brain Res. 749 (2) : 354- 7. Erratum in: Brain Res 757 (2) : 299.

Okada, H., Konemura, T., Maruyama, T (2010) ONO-8539, a novel ep1 receptor antagonist, suppresses bladder hyperactivity via excessive production of prostaglandin e2 (pge2) induced by intravesical instillation of atp in urodynamic evaluation of cynomolgus monkeys. Eur Urol Suppl 9(2):72

Omote K, Yamamoto H, Kawamata T, Nakayama Y, Namiki A. (2002) The effects of intrathecal administration of an antagonist for prostaglandin E receptor subtype EP (1) on mechanical and thermal hyperalgesia in a rat model of postoperative pain. Anesth Analg. 95 (6) : 1708- 12.

Omote K, Kawamata T, Nakayama Y, Kawamata M, Hazama K, Namiki A. (2001) The effects of peripheral administration of a novel selective antagonist for prostaglandin E receptor subtype EP (1), ONO- 8711, in a rat model of postoperative pain. Anesth Analg. 92 (1) : 233- 238.

Rahal S, McVeigh LI, Zhang Y, Guan Y, Breyer MD, Kennedy CR. (2006) Increased severity of renal impairment in nephritic mice lacking the EP1 receptor. Can J Physiol Pharmacol. 84 (8- 9) : 877- 885.

Sarkar S, Hobson AR, Hughes A, Growcott J, Woolf CJ, Thompson DG, Aziz Q. (2003) The prostaglandin E2 receptor- 1 (EP- 1) mediates acid- induced visceral pain hypersensitivity in humans. Gastroenterology. 124 (1) : 18-25.

Samad TA, Sapirstein A, Woolf CJ. (2002) Prostanoids and pain: unraveling mechanisms and revealing therapeutic targets. Trends Mol Med. 2002 Aug; 8 (8) : 390- 6.

Schlötzer-Schrehardt U, Zenkel M, Nüsing R.M. (2002) Expression and Localization of FP and EP Prostanoid. Invest. Ophthalmol. Vis. Sci. 43(5) 1475-1487.

Syriatowicz JP, Hu D, Walker JS, Tracey DJ. (1999) Hyperalgesia due to nerve injury: role of prostaglandins. Neuroscience. 94 (2) : 587- 94.

Teramura, T.; Kawatani, M.; Maruyama, T. (2000) Prostaglandin E1 facilitate primary afferent activity from the urinary bladder in the rat using selective EP1- receptor antagonist (ONO- 8711) . BJU Int 86 (Suppl. 3) : Abst P6.3.19

Watanabe K, Kawamori T, Nakatsugi S, Ohta T, Ohuchida S, Yamamoto H, Maruyama T, Kondo K, Ushikubi F, Narumiya S, Sugimura T, Wakabayashi K. (1999) Role of the prostaglandin E receptor subtype EP1 in colon carcinogenesis. Cancer Res. 59 (20) : 5093- 5096.

Wilbraham D., Masuda T.,Deacon S.,Kuwayama T., Vincent S. (2010) Safety, tolerability and pharmacokinetic of multiple ascending doses of the ep-1 receptor antagonist ono-8539, a potential new and novel therapy to overactive bladder in healthy young and elderly subjects Eur Urol Suppl 9(2):250.

Woodward DF, Regan JW, Lake S, Ocklind A. (1997) The molecular biology and ocular distribution of prostanoid receptors. Surv Ophthalmol. 41 Suppl 2:S15-21.

Zhang M, Ho HC, Sheu TJ, Breyer MD, Flick LM, Jonason JH, Awad HA, Schwarz EM, O'Keefe RJ.J (2011) EP1 (-/-) mice have enhanced osteoblast differentiation and accelerated fracture repair. Bone Miner Res. 26 (4) : 792- 802.

## Claims

**1.** A compound of general formula (I):

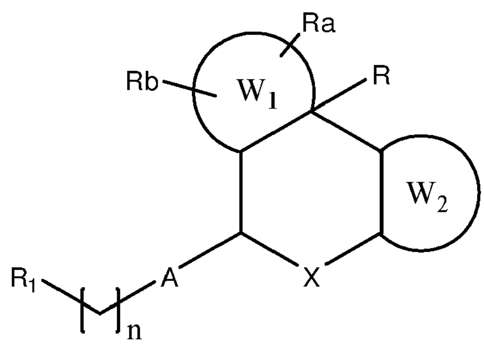

**(I)**

wherein:

**W1** is a 5 or 6-membered saturated heterocyclic ring containing 1 or 2 heteroatoms selected from N, O or S;
**W2** is a benzene ring optionally substituted by one or more $R_2$ and optionally fused to a carbocyclic ring; or a 5 or 6-membered heterocyclic ring containing 1 or 2 heteroatoms selected from N, O or S, optionally substituted by one or more $R_2$;
**R** is a hydrogen; or $C_{1-6}$- alkyl;
**Ra** and **Rb** are independently selected from hydrogen; =O;- OH;- $CH_2OH$;- CO- $C_{1-6}$- alkyl;- $SO_2$- $C_{1-6}$- alkyl; $C_{1-6}$- alkyl;- O- $C_{1-6}$- alkyl or- O- $C_{1-6}$- haloalkyl;
**$R_1$** is a- COOH; a tetrazol; a- $SO_2$- NH- C (=O)- $R_3$; a- C (=O) NH- $SO_2$- $R_4$; a- $SO_2$- OH or a- CN;
**$R_2$** is independently selected from hydrogen; halogen; $C_{1-6}$ alkyl; $C_{1-6}$- haloalkyl; O- $C_{1-6}$- alkyl;- O- $C_{1-6}$- haloalkyl; $C_{3-6}$cycloalkyl;- O- $C_{3-6}$cycloalkyl;- $NR_5SO_2R_6$; phenyl;- $CONH_2$ or- CN;
**$R_3$** and **$R_4$** are independently selected from a hydrogen; $C_{1-6}$- alkyl; an optionally substituted phenyl; or- N $(CH_3)_2$;
**$R_5$** and **$R_6$** are independently selected from a hydrogen; or $C_{1-6}$- alkyl **$R_7$** is selected from a hydrogen; $C_{1-6}$- alkyl; or a- $COR_8$
**$R_8$** is a $C_{1-6}$- alkyl;
**X** is selected from $NR_7$ or O;
**A** is a phenyl optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl; or an heteroaromatic ring or ring system optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, NH- CO- $CH_3$, a- CN or a $C_{3-6}$cycloalkyl optionally substituted by a $C_{1-6}$- alkyl;
**n** is 0, 1 or 2,

and the salts, solvates and prodrugs thereof.

**2.** A compound according to claim 1 having general formula (Ia) :

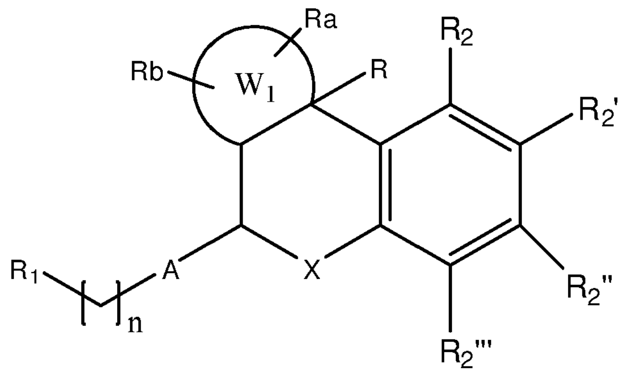

**(Ia)**

wherein **W1**, **X**, **A**, **R**, **Ra**, **Rb**, $R_1$ and n have the same meaning as in claim 1; $\mathbf{R_2}$, $\mathbf{R_{2'}}$, $\mathbf{R_{2''}}$ and $\mathbf{R_{2'''}}$ are independently selected from hydrogen; halogen; $C_{1-6}$ alkyl; $C_{1-6}$- haloalkyl;- O- $C_{1-6}$- alkyl;- O- $C_{1-6}$- haloalkyl; $C_{3-6}$cycloalkyl;- O- $C_{3-6}$cycloalkyl;- $NR_5SO_2R_6$; phenyl;- $CONH_2$ or- CN;
with the proviso that when W1 is a 6- membered saturated heterocyclic ring containing one oxygen as heteroatom atom and $R_1$ is a- COOH, at least one of $\mathbf{R_2}$, $\mathbf{R_{2'}}$, $\mathbf{R_{2''}}$ and $\mathbf{R_{2'''}}$ is different from H; and
with the proviso that when W1 is a 6- membered saturated heterocyclic ring containing one oxygen as heteroatom, $R_1$ is a- COOH and $\mathbf{R_{2'}}$ is methyl, $\mathbf{R_{2''}}$ must be different from methyl or that when W1 is a 6- membered saturated heterocyclic ring containing one oxygen as heteroatom, $R_1$ is a- COOH and $\mathbf{R_{2''}}$ is methyl $\mathbf{R_{2'}}$ must be different from methyl; and
with the proviso that when W1 is a 6- membered saturated heterocyclic ring containing one oxygen as heteroatom, $R_1$ is a- COOH and $\mathbf{R_{2'}}$ is a methoxy group, a methyl group, a tertbutyl group, a chlorine or fluorine or $\mathbf{R_{2'''}}$ is methyl or chlorine, at least A must be substituted.

3. A compound according to claim 2 having general formula (Ia'):

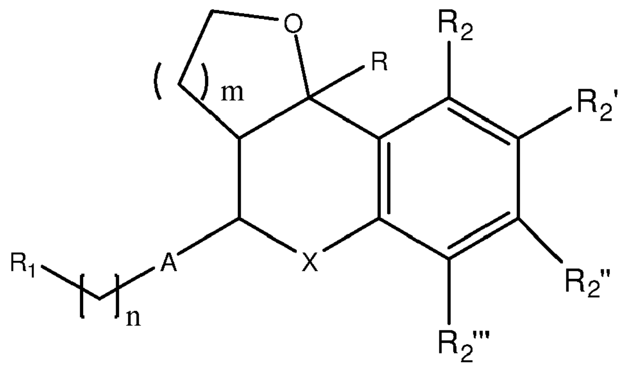

**(Ia')**

wherein **X**, **A**, **R**, $\mathbf{R_1}$, $\mathbf{R_2}$, $\mathbf{R_{2'}}$, $\mathbf{R_{2''}}$ and $\mathbf{R_{2'''}}$ and **n** have the same meaning as in claim 2; **m** is 1 or 2;
with the proviso that when **m**=2 and $R_1$ is a -COOH, at least one of $\mathbf{R_2}$, $\mathbf{R_{2'}}$, $\mathbf{R_{2''}}$ and $\mathbf{R_{2'''}}$ is different from H; and
with the proviso that when **m**=2, $R_1$ is a -COOH and $\mathbf{R_{2'}}$ is methyl, $\mathbf{R_{2''}}$ must be different from methyl or that when **m**=2, $R_1$ is a -COOH and $\mathbf{R_{2''}}$ is methyl, $\mathbf{R_{2'}}$ must be different from methyl; and
with the proviso that when **m**=2, $R_1$ is a -COOH and $\mathbf{R_{2'}}$ is a methoxy group, a methyl group, a tertbutyl group, a chlorine or fluorine or $\mathbf{R_{2'''}}$ is methyl or chlorine,
at least A must be substituted.

4. A compound according to claim 1 having general formula (Ib):

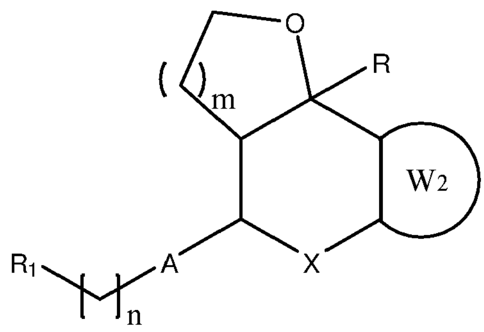

**(Ib)**

wherein **X**, **A**, **R**, $\mathbf{R_1}$ and n have the same meaning as in claim 1; **m** is 1 or 2; and **W2** is a 5 or 6-membered heterocyclic ring containing 1 or 2 heteroatoms selected from N, O or S, optionally substituted by one or more $\mathbf{R_2}$ where $\mathbf{R_2}$ has the same meaning as in claim 1.

**5.** A compound according to claim 1 having general formula (Ic) :

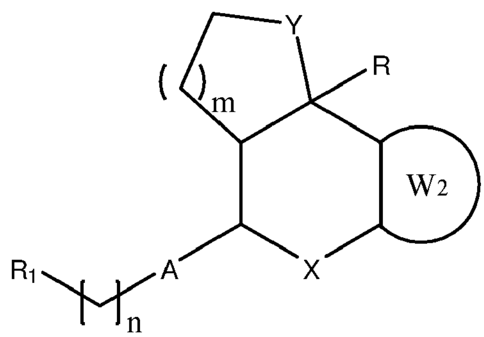

**(Ic)**

wherein **W2**, **X**, **A**, **R**, $\mathbf{R_1}$ and n have the same meaning as in claim 1; **m** is 1 or 2and **Y** is S or $NR_9$ where $R_9$ is hydrogen or $C_{1-6}$- alkyl.

**6.** A compound according to any of claims 1 to 5 where $\mathbf{R_1}$ is -COOH.

**7.** A compound according to any of claims 1 to 5 where **A** is a phenyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl; a pyridinyl group optionally substituted by one or more substituents selected from $C_{1-6}$- alkyl, halogen, $C_{1-6}$- haloalkyl, - O- $C_{1-6}$- alkyl, -O- $C_{1-6}$- haloalkyl, -NH- CO-$CH_3$, a- CN or a $C_{3-6}$cycloalkyl optionally substituted by a $C_{1-6}$- alkyl; a pyrazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH-CO- $CH_3$, a- CN or a $C_{3-6}$cycloalkyl optionally substituted by a $C_{1-6}$- alkyl; an imidazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl; or a thiazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl.

**8.** A compound according to any of claims 1 to 5 where **X** is NR, where $R_7$ is a hydrogen.

**9.** A compound according to claim 1 or 5 where **W2** is a benzene ring optionally substituted by one or more $\mathbf{R_2}$ where

$R_2$ has the same meaning as in claim 1.

**10.** A compound according to any of claims 1 to 9 where n is 0.

**11.** A compound according to claim 1 selected from:

- 4- ((4aR, 5S, 10bR)- 9- bromo- 3, 4, 4a, 5, 6, 10b- hexahydro- 2H- pyrano [3, 2- c] quinolin- 5- yl) benzoic acid;
- 4- ((4aR, 5R, 10bR)- 9- bromo- 3, 4, 4a, 5, 6, 1 0b- hexahydro- 2H- pyrano [3, 2- c] quinolin- 5- yl) benzoic acid;
- 4- ((4aR, 5S, 10bR)- 3, 4, 4a, 5, 6, 10b- hexahydro- 9- methyl- 2H- pyrano [3, 2- c] quinolin- 5- yl) benzoic acid;
- 4- ((4aR, 5R, 10bR)- 3, 4, 4a, 5, 6, 1 0b- hexahydro- 9- methyl- 2H- pyrano [3, 2- c] quinolin- 5- yl) benzoic acid;
- 4- ((4aR, 5R, 10bR)- 3, 4, 4a, 5, 6, 10b- hexahydro- 9- methoxy- 2H- pyrano [3, 2- c] quinolin- 5- yl)- 3- methylbenzoic acid;
- 4- ((4aR, 5R, 10bR)- 3, 4, 4a, 5, 6, 1 0b- hexahydro- 9- methyl- 2H- pyrano [3, 2- c] quinolin- 5- yl)- 3- methylbenzoic acid;
- 4- ((4aR, 5R, 10bR)- 9- chloro- 3, 4, 4a, 5, 6, 1 0b- hexahydro- 2H- pyrano [3, 2- c] quinolin- 5- yl)- 3- methylbenzoic acid;
- 4- ((4aR, 5S, 10bR)- 3, 4, 4a, 5, 6, 10b- hexahydro- 9- methoxy- 2H- pyrano [3, 2- c] quinolin- 5- yl)- 3- methylbenzoic acid;
- 4- ((4aR, 5S, 10bR)- 3, 4, 4a, 5, 6, 10b- hexahydro- 9- methoxy- 2H- pyrano [3, 2- c] quinolin- 5- yl)- 3- methylbenzoic acid (enantiomer 1) ;
- 4- ((4aR, 5S, 10bR)- 3, 4, 4a, 5, 6, 1 0b- hexahydro- 9- methoxy- 2H- pyrano [3, 2- c] quinolin- 5- yl)- 3- methylbenzoic acid (enantiomer 2) ;
- 4- ((4aR, 5S, 10bR)- 3, 4, 4a, 5, 6, 10b- hexahydro- 9- methyl- 2H- pyrano [3, 2- c] quinolin- 5- yl)- 3- methylbenzoic acid;
- 4- ((4aR, 5S, 10bR)- 3, 4, 4a, 5, 6, 10b- hexahydro- 9- methyl- 2H- pyrano [3, 2- c] quinolin- 5- yl)- 3- methylbenzoic acid (enantiomer 1) ;
- 4- ((4aR, 5S, 10bR)- 3, 4, 4a, 5, 6, 1 0b- hexahydro- 9- methyl- 2H- pyrano [3, 2- c] quinolin- 5- yl)- 3- methylbenzoic acid (enantiomer 2) ;
- 4- ((4aR, 5S, 10bR)- 9- chloro- 3, 4, 4a, 5, 6, 10b- hexahydro- 2H- pyrano [3, 2- c] quinolin- 5- yl)- 3- methylbenzoic acid;
- 4- ((4aR, 5S, 10bR)- 9- chloro- 3, 4, 4a, 5, 6, 10b- hexahydro- 2H- pyrano [3, 2- c] quinolin- 5- yl)- 3- methylbenzoic acid (enantiomer 1) ;
- 4- ((4aR, 5S, 10bR)- 9- chloro- 3, 4, 4a, 5, 6, 10b- hexahydro- 2H- pyrano [3, 2- c] quinolin- 5- yl)- 3- methylbenzoic acid (enantiomer 2) ;
- 4- (8- bromo- 2, 3, 3a, 4, 5, 9b- hexahydrofuro [3, 2- c] quinolin- 4- yl) benzoic acid;

**12.** A compound according to any of the claims **¡Error! No se encuentra el origen de la referencia.** to 11 for use as a medicament.

**13.** A compound according to any of claims 1 to 11 for use in the treatment and/or prophylaxis of diseases or disorders mediated by the EP1 receptor.

**14.** A compound according to claim 13 where the disease or disorders comprises inflammatory related pain including low back and neck pain, skeletal pain, post-partum pain, toothache, sprains and straits, myositis, neuralgia, synovitis, arthritis, including rheumatoid arthritis, degenerative joint diseases, gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases.

**15.** A compound according to claim 13 where the disease or disorders comprises pain, inflammatory related pain

including low back and neck pain, skeletal pain, post-partum pain, toothache, sprains and straits, myositis, neuralgia, synovitis, arthritis, including rheumatoid arthritis, degenerative joint diseases, gout and ankylosing spondylitis, bursitis, burns including radiation and corrosive chemical injuries and sunburns; postoperative pain; neuropathic pain; visceral pain; tension headache; cluster headaches; migraine.

**16.** Pharmaceutical composition comprising at least one compound according to any of claims 1-11 and at least one pharmaceutically acceptable carrier, additive, adjuvant or vehicle.

Europäisches Patentamt
European Patent Office
Office européen des brevets

# EUROPEAN SEARCH REPORT

Application Number
EP 12 38 2131

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHUQING ZHOU ET AL: "Stereoselective Synthesis of Pyrano[3,2-c]- and Furano[3,2-c]quinolines: Samarium Diiodide-Catalyzed One-Pot Aza-Diels-Alder Reactions", EUROPEAN JOURNAL OF ORGANIC CHEMISTRY, vol. 2007, no. 31, 1 November 2007 (2007-11-01), pages 5265-5269, XP55029950, ISSN: 1434-193X, DOI: 10.1002/ejoc.200700288 * examples 3g, 4g * | 1-3,7-10 | INV. C07D491/04 A61K31/4355 A61K31/436 A61P1/00 A61P7/00 A61P9/00 A61P13/00 A61P19/00 A61P25/00 A61P31/00 A61P35/00 A61P37/00 |
| X | WO 2005/016255 A2 (LIGAND PHARM INC [US]; MICHELLYS PIERRE [US]; CHEN JYUN-HUNG [US]; MEY) 24 February 2005 (2005-02-24) * examples 1-10, 12, 19-22, 24-26,33-43 * * claims 1-27 * | 1-16 | |
| X | WO 98/27093 A1 (LILLY CO ELI [US]; MENDOZA JOSE S [US]) 25 June 1998 (1998-06-25) * page 46; example 3 * * claims 1-5 * | 1-3, 7-10,12 | TECHNICAL FIELDS SEARCHED (IPC) |
| X | BAUDELLE R ET AL: "Parallel Synthesis of Polysubstituted Tetrahydroquinolines", TETRAHEDRON, ELSEVIER SCIENCE PUBLISHERS, AMSTERDAM, NL, vol. 54, no. 16, 16 April 1998 (1998-04-16), pages 4125-4140, XP004162185, ISSN: 0040-4020, DOI: 10.1016/S0040-4020(98)00140-9 * page 4130; compound 8 * | 1-3,7-10 | C07D A61K A61P |
| | -/-- | | |

The present search report has been drawn up for all claims

| Place of search | Date of completion of the search | Examiner |
|---|---|---|
| Munich | 18 June 2012 | Marzi, Elena |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 38 2131

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online] CHEMICAL ABSTRACTS SERVICE, COLUMBUS, OHIO, US; JIA, ZHONG: "Synthesis of diaryl tetrahydroquinoline compounds", XP002677859, retrieved from STN Database accession no. 2011:1053366 * abstract * * RN: 1330059-19-2; 1330059-21-6, 1330059-22-7, 1330059-24-9, 1330059-28-3 * & CN 102 153 561 A (PEOP. REP. CHINA) 17 August 2011 (2011-08-17) ----- | 1-3,7-10 | |
| X | DATABASE CA [Online] CHEMICAL ABSTRACTS SERVICE, COLUMBUS, OHIO, US; March 2012 (2012-03), JIA, ZHONG ET AL: "Radical cation salts induced aza-diels-alder reaction: synthesis of hexahydrofuro[3,2-c]-quinoline derivatives", XP002677860, retrieved from STN Database accession no. 2012:487414 * abstract * * RN:1372402-11-3 * & JIA, ZHONG ET AL: "Radical cation salts induced aza-diels-alder reaction: synthesis of hexahydrofuro[3,2-c]-quinoline derivatives", LETTERS IN ORGANIC CHEMISTRY , 9(3), 221-224 CODEN: LOCEC7; ISSN: 1570-1786, 2012, ----- | 1-3,7-10 | TECHNICAL FIELDS SEARCHED (IPC) |

-/--

The present search report has been drawn up for all claims

2

| Place of search | Date of completion of the search | Examiner |
|---|---|---|
| Munich | 18 June 2012 | Marzi, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 38 2131

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | SCHIEMANN K ET AL: "The discovery and optimization of hexahydro-2H-pyrano[3,2-c]quinolines (HHPQs) as potent and selective inhibitors of the mitotic kinesin-5", BIOORGANIC & MEDICINAL CHEMISTRY LETTERS, PERGAMON, ELSEVIER SCIENCE, GB, vol. 20, no. 5, 1 March 2010 (2010-03-01), pages 1491-1495, XP026911287, ISSN: 0960-894X, DOI: 10.1016/J.BMCL.2010.01.110 [retrieved on 2010-01-25] * page 1493; example 38; table 2 * * abstract * | 1,6-10, 12-16 | |
| X | WO 2005/063735 A1 (MERCK PATENT GMBH [DE]; SCHIEMANN KAI [DE]; ANZALI SOHEILA [DE]; DROSD) 14 July 2005 (2005-07-14) * examples 37, 48, 53, 99, 189, 190, 375, 397, 465 * * page 1, lines 1-10 * * claim 1 * | 1-16 | TECHNICAL FIELDS SEARCHED (IPC) |
| X | WO 2005/105802 A1 (TAKEDA PHARMACEUTICAL [JP]; KAJINO MASAHIRO [JP]; HIRD NICHOLAS WILLIA) 10 November 2005 (2005-11-10) * page 90; example 10 * | 1-3,5, 7-10 | |
| X | WO 2006/002726 A1 (MERCK PATENT GMBH [DE]; SCHIEMANN KAI [DE]; BRUGE DAVID [DE]; BUCHSTAL) 12 January 2006 (2006-01-12) * claim 1 * * examples 1107, 1119, 1189 * * page 1, lines 1-10 * | 1-3, 7-10, 12-16 | |
| | -/-- | | |

The present search report has been drawn up for all claims

2

| Place of search | Date of completion of the search | Examiner |
|---|---|---|
| Munich | 18 June 2012 | Marzi, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 38 2131

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2008/098978 A2 (GLAXO GROUP LTD [GB]; EATHERTON ANDREW JOHN [GB]; GIBLIN GERARD MARTIN) 21 August 2008 (2008-08-21)<br>* claims 1-11 *<br>----- | 1-16 | |
| A | WO 2008/006794 A1 (GLAXO GROUP LTD [GB]; GIBLIN GERARD MARTIN PAUL [GB]; GIBSON MAIRI [GB) 17 January 2008 (2008-01-17)<br>* claims 1-11 *<br>----- | 1-16 | |
| | | | TECHNICAL FIELDS SEARCHED (IPC) |

The present search report has been drawn up for all claims

2

| Place of search | Date of completion of the search | Examiner |
|---|---|---|
| Munich | 18 June 2012 | Marzi, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 38 2131

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2012

| Patent document cited in search report | Publication date | Patent family member(s) | Publication date |
|---|---|---|---|
| WO 2005016255 A2 | 24-02-2005 | NONE | |
| WO 9827093 A1 | 25-06-1998 | AU 5797098 A | 15-07-1998 |
| | | CA 2273884 A1 | 25-06-1998 |
| | | EP 0946556 A1 | 06-10-1999 |
| | | JP 2001508050 A | 19-06-2001 |
| | | WO 9827093 A1 | 25-06-1998 |
| WO 2005063735 A1 | 14-07-2005 | AT 413393 T | 15-11-2008 |
| | | AT 507220 T | 15-05-2011 |
| | | AU 2004309028 A1 | 14-07-2005 |
| | | BR PI0417838 A | 17-04-2007 |
| | | CA 2550350 A1 | 14-07-2005 |
| | | DK 1761515 T3 | 16-02-2009 |
| | | DK 2033959 T3 | 25-07-2011 |
| | | EP 1761515 A1 | 14-03-2007 |
| | | EP 2033959 A1 | 11-03-2009 |
| | | ES 2315727 T3 | 01-04-2009 |
| | | JP 2007515419 A | 14-06-2007 |
| | | PT 1761515 E | 11-02-2009 |
| | | PT 2033959 E | 29-07-2011 |
| | | SI 1761515 T1 | 28-02-2009 |
| | | SI 2033959 T1 | 31-08-2011 |
| | | US 2007203167 A1 | 30-08-2007 |
| | | WO 2005063735 A1 | 14-07-2005 |
| WO 2005105802 A1 | 10-11-2005 | AT 416176 T | 15-12-2008 |
| | | AU 2005238388 A1 | 10-11-2005 |
| | | BR PI0510365 A | 06-11-2007 |
| | | CA 2565018 A1 | 10-11-2005 |
| | | CN 1976932 A | 06-06-2007 |
| | | CR 8755 A | 17-01-2007 |
| | | DK 1740584 T3 | 30-03-2009 |
| | | EP 1740584 A1 | 10-01-2007 |
| | | EP 2080760 A1 | 22-07-2009 |
| | | ES 2318483 T3 | 01-05-2009 |
| | | HK 1099284 A1 | 07-08-2009 |
| | | HR 20090121 T3 | 30-04-2009 |
| | | JP 4361566 B2 | 11-11-2009 |
| | | JP 2007534638 A | 29-11-2007 |
| | | JP 2009256362 A | 05-11-2009 |
| | | KR 20070015598 A | 05-02-2007 |
| | | MY 143370 A | 29-04-2011 |
| | | NZ 550983 A | 27-08-2010 |
| | | PT 1740584 E | 10-03-2009 |
| | | RU 2384571 C2 | 20-03-2010 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 12 38 2131

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2012

| Patent document cited in search report | Publication date | Patent family member(s) | Publication date |
|---|---|---|---|
| | | SI 1740584 T1 | 30-06-2009 |
| | | US 2008039452 A1 | 14-02-2008 |
| | | US 2009258893 A1 | 15-10-2009 |
| | | US 2009270625 A1 | 29-10-2009 |
| | | US 2009275568 A1 | 05-11-2009 |
| | | WO 2005105802 A1 | 10-11-2005 |
| | | ZA 200609261 A | 27-08-2008 |
| WO 2006002726 A1 | 12-01-2006 | AR 049654 A1 | 23-08-2006 |
| | | AU 2005259676 A1 | 12-01-2006 |
| | | BR PI0512784 A | 08-04-2008 |
| | | CA 2572350 A1 | 12-01-2006 |
| | | CN 1976936 A | 06-06-2007 |
| | | DE 102004031656 A1 | 19-01-2006 |
| | | EP 1778694 A1 | 02-05-2007 |
| | | JP 2008505136 A | 21-02-2008 |
| | | KR 20070037585 A | 05-04-2007 |
| | | US 2009030028 A1 | 29-01-2009 |
| | | WO 2006002726 A1 | 12-01-2006 |
| | | ZA 200700819 A | 30-01-2008 |
| WO 2008098978 A2 | 21-08-2008 | EP 2129670 A2 | 09-12-2009 |
| | | JP 2010518146 A | 27-05-2010 |
| | | US 2010056527 A1 | 04-03-2010 |
| | | WO 2008098978 A2 | 21-08-2008 |
| WO 2008006794 A1 | 17-01-2008 | EP 2041126 A1 | 01-04-2009 |
| | | JP 2009543837 A | 10-12-2009 |
| | | US 2009281152 A1 | 12-11-2009 |
| | | WO 2008006794 A1 | 17-01-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BURGER.** Medicinal Chemistry and Drug Discovery. Wiley, 2001
- Design and Applications of Prodrugs. Harwood Academic Publishers, 1985
- **T.W. GREENE ; P.G.M. WUTS.** protective Groups in Organic Chemistry. John Wiley&Sons, 1999
- **ABE T ; KUNZ A ; SHIMAMURA M ; ZHOU P ; ANRATHER J ; LADECOLA C.** The neuroprotective effect of prostaglandin E2 EP1 receptor inhibition has a wide therapeutic window, is sustained in time and is not sexually dimorphic. *J Cereb Blood Flow Metab.,* 2009, vol. 29 (1), 66-72
- **ASBÓTH G ; PHANEUF S ; EUROPE-FINNER GN ; TÓTH M ; BERNAL AL.** Prostaglandin E2 activates phospholipase C and elevates intracellular calcium in cultured myometrial cells: involvement of EP1 and EP3 receptor subtypes. *Endocrinology,* 1996, vol. 137 (6), 2572-9
- **BABA H ; KOHNO T ; MOORE KA ; WOOLF CJ.** Direct activation of rat spinal dorsal horn neurons by prostaglandin E2. *The Journal of Neuroscience,* 2001, vol. 21 (5), 1750-1756
- **BREYER MD ; BREYER RM.** Prostaglandin receptors: their role in regulating renal function. *Curr Opin Nephrol Hypertens,* January 2000, vol. 9 (1), 23-9
- **CANDELARIO-JALIL E ; SLAWIK H ; RIDELIS I ; WASCHBISCH A ; AKUNDI RS ; HÜLL M ; FIEBICH BL.** Regional distribution of the prostaglandin E2 receptor EP1 in the rat brain: accumulation in Purkinje cells of the cerebellum. *J Mol Neurosci.,* 2005, vol. 27 (3), 303-10
- Prostanoid Receptors. **COLEMAN, R. A.** IUPHAR compendium of receptor characterization and classification. 2000, 338-353
- **DIRIG DM ; YAKSH TL.** In vitro prostanoid release from spinal cord following peripheral inflammation: effects of substance P, NMDA and capsaicin. *Br J Pharmacol.,* 1999, vol. 126 (6), 1333-40
- **DURRENBERGER PF ; FACER P ; CASULA MA ; YIANGOU Y ; GRAY RA ; CHESSELL IP ; DAY NC ; COLLINS SD ; BINGHAM S ; WILSON AW.** Prostanoid receptor EP1 and Cox-2 in injured human nerves and a rat model of nerve injury: a time-course study. *BMC Neurol.,* 2006, vol. 4 (6), 1
- **GIBLIN GM ; BIT RA ; BROWN SH ; CHAIGNOT HM ; CHOWDHURY A ; CHESSELL IP ; CLAYTON NM ; COLEMAN T ; HALL A ; HAMMOND B.** The discovery of 6-[2-(5-chloro-2-{[(2,4-difluorophenyl)methyl]oxy}phenyl)-1-cyclopenten-1-yl]-2-pyridinecarboxylic acid, GW848687X, a potent and selective prostaglandin EP1 receptor antagonist for the treatment of inflammatory pain. *Bioorg Med Chem Lett.,* 2007, vol. 17 (2), 385-9
- **GUAY J. ; BATEMAN, K. ; GORDON R. ; MANCINI J. ; RIENDEAU D.** Carrageenan-induced paw edema in rat elicits a predominant prostaglandin E2 (PGE2) response in the central nervous system associated with the induction of microsomal PGE2 synthase-1. *J. Biol Chem,* 2004, vol. 279, 24866-24872
- **HALL, A. ; BILLINTON A. ; GIBLIN G.M.** EP1 antagonists for the treatment of inflammatory pain. *Curr Opin. Drug Discov. Devel.,* 2007, vol. 10, 597-612
- **HALL A ; BROWN SH ; BUDD C ; CLAYTON NM ; GIBLIN GM ; GOLDSMITH P ; HAYHOW TG ; HURST DN ; NAYLOR A ; ANTHONY RAWLINGS D.** Discovery of GSK345931 A: An EP(1) receptor antagonist with efficacy in preclinical models of inflammatory pain. *Bioorg Med Chem Lett.,* 2009, vol. 19 (2), 497-501
- **HÖNEMANN CW ; HEYSE TJ ; MÖLLHOFF T ; HAHNENKAMP K ; BERNING S ; HINDER F ; LINCK B ; SCHMITZ W ; VAN AKEN H.** The inhibitory effect of bupivacaine on prostaglandin E(2) (EP(1)) receptor functioning: mechanism of action. *Anesth Analg.,* 2001, vol. 93 (3), 628-634
- **JOHANSSON T ; NARUMIYA S ; ZEILHOFER HU.** Contribution of peripheral versus central EP1 prostaglandin receptors to inflammatory pain. *Neurosci Lett.,* 2011, vol. 495 (2), 98-101
- **KAWAHARA H ; SAKAMOTO A ; TAKEDA S ; ONODERA H ; IMAKI J ; OGAWA R.** A prostaglandin E2 receptor subtype EP1 receptor antagonist (ONO-8711) reduces hyperalgesia, allodynia, and c-fos gene expression in rats with chronic nerve constriction. *Anesth Analg.,* 2001, vol. 93 (4), 1012-7
- **LEE T ; HEDLUND P ; NEWGREEN D ; ANDERSSON KE.** Urodynamic effects of a novel EP(1) receptor antagonist in normal rats and rats with bladder outlet obstruction. *J Urol.,* 2007, vol. 177 (4), 1562-1567

- **LEE C.M ; GENETOS DC ; YOU Z ; YELLOWLEY CE.** Hypoxia regulates PGE(2) release and EP1 receptor expression in osteoblastic cells. *J Cell Physiol.,* 2007, vol. 212 (1), 182-188
- **LI X ; CUDABACK E ; KEENE CD ; BREYER RM ; MONTINE TJ.** Suppressed microglial E prostanoid receptor 1 signaling selectively reduces tumor necrosis factor alpha and interleukin 6 secretion from toll-like receptor 3 activation. *Glia,* 2011, vol. 59 (4), 569-576
- **LIN CR ; AMAYA F ; BARRETT L ; WANG H ; TAKADA J ; SAMAD TA ; WOOLF CJ.** Prostaglandin E2 receptor EP4 contributes to inflammatory pain hypersensitivity. *J Pharmacol Exp Ther.,* 2006, vol. 319 (3), 1096-103
- **MA W ; EISENACH JC.** Four PGE2 EP receptors are up-regulated in injured nerve following partial sciatic nerve ligation. *Exp Neurol.,* 2003, vol. 183 (2), 581-92
- **MIKI, T. ; MATSUNAMI, M. ; OKADA, H. ; MATSUYA, H. ; KAWABATA, A.** ONO-8130, an EP1 antagonist, strongly attenuates cystitis-related bladder pain caused by cyclophosphamide in mice. *J Pharmacol Sci,* 2010, vol. 112 (1), 1-2
- **MINAMI T ; UDA R. ; HORIGUCHI S. ; ITO S. ; HYODO M. ; HAYAISHI O.** Allodynia evoked by intrathecal administration fo prostaglandin E2 to conscious mice. *Pain,* 1994, vol. 57, 217-223
- **MINAMI T ; NAKANO H ; KOBAYASHI T ; SUGIMOTO Y ; USHIKUBI F ; ICHIKAWA A ; NARUMIYA S ; ITO S.** Characterization of EP receptor subtypes responsible for prostaglandin E2-induced pain responses by use of EP1 and EP3 receptor knockout mice. *Br J Pharmacol.,* 2001, vol. 133 (3), 438-44
- **MIZUGUCHI S ; OHNO T ; HATTORI Y ; AE T ; MINAMINO T ; SATOH T ; ARAI K ; SAEKI T ; HAYASHI I ; SUGIMOTO Y.** Roles of prostaglandin E2-EP1 receptor signaling in regulation of gastric motor activity and emptying. *Am J Physiol Gastrointest Liver Physiol.,* 2010, vol. 299 (5), G1078-1086
- **MORIYAMA T ; HIGASHI T ; TOGASHI K ; LIDA T ; SEGI E ; SUGIMOTO Y ; TOMINAGA T ; NARUMIYA S ; TOMINAGA M.** Sensitization of TRPV1 by EP1 and IP reveals peripheral nociceptive mechanism of prostaglandins. *Mol Pain.,* 2005, vol. 1, 3
- **NAKAYAMA Y ; OMOTE K ; NAMIKI A.** Role of prostaglandin receptor EP1 in the spinal dorsal horn in carrageenan-induced inflammatory pain. *Anesthesiology,* 2002, vol. 97, 1254-62
- **NAKAYAMA Y ; OMOTE K ; KAWAMATA T ; NAMIKI A.** Role of prostaglandin receptor subtype EP1 in prostaglandin E2-induced nociceptive transmission in the rat spinal dorsal horn. *Brain Res.,* 2004, vol. 1010 (1-2), 62-8
- **NARUMIYA S. ; SUGIMOTO Y. ; USHIKUBI F.** Prostanoid receptors: structures, properties, and functions. *Physiol Rev.,* 1999, vol. 79, 1193-1226
- **NIHO N ; MUTOH M ; KITAMURA T ; TAKAHASHI M ; SATO H ; YAMAMOTO H ; MARUYAMA T ; OHUCHIDA S ; SUGIMURA T ; WAKABAYASHI K.** Suppression of azoxymethane-induced colon cancer development in rats by a prostaglandin E receptor EP1-selective antagonist. *Cancer Sci.,* 2005, vol. 96 (5), 260-264
- **OIDDA H. ; NAMBA T. ; SUGIMOTO Y. ; USHIKUBI F. ; OHISHI H. ; ICHIKAWA A. et al.** In situ hybridization studies of prostacyclin receptor mRNA expression in various mouse organs. *Br J Pharmacol,* 1995, vol. 116, 2828-2837
- **OKA T ; OKA K ; SAPER CB.** Contrasting effects of E type prostaglandin (EP) receptor agonists on core body temperature in rats. *Brain Res.,* 2003, vol. 968 (2), 256-262
- **OKA T ; HOSOI M ; OKA K ; HORI T.** Biphasic alteration in the trigeminal nociceptive neuronal responses after intracerebroventricular injection of prostaglandin E2 in rats. *Brain Res.,* 1997, vol. 749 (2), 354-7
- *Brain Res,* vol. 757 (2), 299
- **OKADA, H. ; KONEMURA, T. ; MARUYAMA, T.** ONO-8539, a novel ep1 receptor antagonist, suppresses bladder hyperactivity via excessive production of prostaglandin e2 (pge2) induced by intravesical instillation of atp in urodynamic evaluation of cynomolgus monkeys. *Eur Urol,* 2010, vol. 9, 72
- **OMOTE K ; YAMAMOTO H ; KAWAMATA T ; NAKAYAMA Y ; NAMIKI A.** The effects of intrathecal administration of an antagonist for prostaglandin E receptor subtype EP(1) on mechanical and thermal hyperalgesia in a rat model of postoperative pain. *Anesth Analg.,* 2002, vol. 95 (6), 1708-12
- **OMOTE K ; KAWAMATA T ; NAKAYAMA Y ; KAWAMATA M ; HAZAMA K ; NAMIKI A.** The effects of peripheral administration of a novel selective antagonist for prostaglandin E receptor subtype EP(1), ONO-8711, in a rat model of postoperative pain. *Anesth Analg.,* 2001, vol. 92 (1), 233-238
- **RAHAL S ; MCVEIGH LI ; ZHANG Y ; GUAN Y ; BREYER MD ; KENNEDY CR.** Increased severity of renal impairment in nephritic mice lacking the EP1 receptor. *Can J Physiol Pharmacol.,* 2006, vol. 84 (8-9), 877-885
- **SARKAR S ; HOBSON AR ; HUGHES A ; GROWCOTT J ; WOOLF CJ ; THOMPSON DG ; AZIZ Q.** The prostaglandin E2 receptor-1 (EP-1) mediates acid-induced visceral pain hypersensitivity in humans. *Gastroenterology,* 2003, vol. 124 (1), 18-25
- **SAMAD TA ; SAPIRSTEIN A ; WOOLF CJ.** Prostanoids and pain: unraveling mechanisms and revealing therapeutic targets. *Trends Mol Med.,* 2002, vol. 8 (8), 390-6

- **SCHLÖTZER-SCHREHARDT U ; ZENKEL M ; NÜSING R.M.** Expression and Localization of FP and EP Prostanoid. *Invest. Ophthalmol. Vis. Sci.,* 2002, vol. 43 (5), 1475-1487
- **SYRIATOWICZ JP ; HU D ; WALKER JS ; TRACEY DJ.** Hyperalgesia due to nerve injury: role of prostaglandins. *Neuroscience,* 1999, vol. 94 (2), 587-94
- **TERAMURA, T. ; KAWATANI, M. ; MARUYAMA, T.** Prostaglandin E1 facilitate primary afferent activity from the urinary bladder in the rat using selective EP1-receptor antagonist (ONO-8711. *BJU Int,* 2000, vol. 86 (3
- **WATANABE K ; KAWAMORI T ; NAKATSUGI S ; OHTA T ; OHUCHIDA S ; YAMAMOTO H ; MARUYAMA T ; KONDO K ; USHIKUBI F ; NARUMIYA S.** Role of the prostaglandin E receptor subtype EP1 in colon carcinogenesis. *Cancer Res.,* 1999, vol. 59 (20), 5093-5096
- **WILBRAHAM D. ; MASUDA T. ; DEACON S. ; KUWAYAMA T. ; VINCENT S.** Safety, tolerability and pharmacokinetic of multiple ascending doses of the ep-1 receptor antagonist ono-8539, a potential new and novel therapy to overactive bladder in healthy young and elderly subjects. *Eur Urol,* 2010, vol. 9 (2), 250
- **WOODWARD DF ; REGAN JW ; LAKE S ; OCKLIND A.** The molecular biology and ocular distribution of prostanoid receptors. *Surv Ophthalmol.,* 1997, vol. 41 (2), 15-21
- **ZHANG M ; HO HC ; SHEU TJ ; BREYER MD ; FLICK LM ; JONASON JH ; AWAD HA ; SCHWARZ EM ; O'KEEFE RJ.J.** EP1(-/-) mice have enhanced osteoblast differentiation and accelerated fracture repair. *Bone Miner Res.,* 2011, vol. 26 (4), 792-802